# EUROPEAN PATENT APPLICATION

(11) **EP 4 711 461 A1**
(43) Date of publication of application: **18.03.2026**
(21) Application number: 24806511.2
(22) Date of filing: 11.05.2024
(51) Int. Cl.: C12N 15/12, C12N 15/113, C12N 15/864, A61K 48/00, A61K 31/7088, A61P 25/00, A61P 21/00, C12Q 1/70

(54) **VIRAL VECTOR CARRYING SMN GENE EXPRESSION CASSETTE AND USE THEREOF**

(30) Priority: 12.05.2023 CN 202310536569
(71) Applicant: Genecombio Ltd., Shanghai 200131 (CN); Chang, Bingsheng, Shanghai 200131 (CN)
(72) Inventor: CHENG, Deqin, Shanghai 200131 (CN); CHANG, Bingsheng, Shanghai 200131 (CN)
(74) Representative: Ostertag & Partner Patentanwälte mbB
(86) International application number: PCT/CN2024/092634
(87) International publication number: WO 2024/235159

(57) **Abstract**

Disclosed are an SMN gene expression cassette, a viral vector carrying the SMN gene expression cassette, and a use of the viral vector in treating spinal muscular atrophy, relating to the field of gene therapy. Codon optimization is carried out on an SMN1 gene in the gene expression cassette, so that the expression level of an SMN protein after SMN1 gene transduction is increased to effectively reduce the clinical dosage. Moreover, a regulatory element such as an miRNA target sequence is also introduced into the gene expression cassette to mitigate an immune response of an organism to an adeno-associated virus vector and a transgenic product, so as to reduce the safety problems possibly caused by gene therapy.

## Description

### TECHNICAL FIELD

The present application relates to the field of gene therapy, and specifically to a gene expression cassette comprising a polynucleotide encoding a human SMN protein, a viral vector carrying the gene expression cassette, and use thereof in the treatment of spinal muscular atrophy.

### BACKGROUND

Spinal muscular atrophy (SMA) is a severe neuromuscular disease characterized by the loss of spinal motor neurons, which leads to muscle weakness and atrophy. The incidence rate of SMA is about 1/6000 to 1/10,000, which is a major genetic cause of infant death worldwide. SMA exhibits diverse clinical manifestations and a wide range of onset ages. It is classified into five types (Type 0, Type 1, Type 2, Type 3, and Type 4) based on the age of onset and clinical features. The prevalence of Types 1-3 ranges from 2.78 to 6.56 per 100,000 population, while the prevalence of Type 4 SMA is 0.32 per 100,000 population. Type 1 is the most common and severe form (also known as Werdnig-Hoffmann disease, acute SMA, or infantile SMA), typically onset within 6 months after birth. Subjects with this type often die of respiratory failure within 2 years after birth, ranking it as the top fatal genetic disease among children under 2 years old. Respiratory failure caused by a disease affecting the respiratory system is the most common cause of death. Type 2 is the intermediate form (also called adolescent SMA or chronic SMA), with onset between 6 and 18 months after birth. Type 3 is the mild form (also known as Kugelberg-Welander disease or Wohlfart-Kugelberg-Welander disease), generally onset after 18 months of age. Type 4, also referred to as adult SMA, usually occurs after the age of 35. In fact, SMA has a broad clinical spectrum, and there is overlap between different subtypes.

The pathogenesis of SMA is associated with defects in the survival motor neuron (SMN) gene, which expresses a SMN protein essential for the survival of motor neurons. The SMN gene is located at 5q13.2 and has two highly homologous copies, SMN1 and SMN2, with only 5 base differences between them. Two bases are located in exons 7 and 8, while the other three bases are located in introns 6 and 7. The SMN protein is mainly encoded by the SMN1 gene. Homozygous deletions or point mutations in exons 7 or exons 7 and 8 of SMN1 result in the loss of SMN protein expression, leading to the degeneration of α-motor neurons in the anterior horn of the spinal cord, reduced neurotransmitter release, and the failure of effective transmission of nerve signals to skeletal muscles for contraction. Ultimately, this leads to the typical clinical manifestations of SMA subjects, such as muscle atrophy, weakness, and paralysis. Studies have shown that only approximately 10%-15% of the SMN2 gene can be expressed as full-length SMN protein, hence it is also known as the compensatory gene for SMA. The copy numbers of the SMN1 and SMN2 genes are variable in the population, and the copy number of the SMN2 gene is negatively correlated with the severity of SMA. The more SMN2 gene copies an SMA patient has, the later the onset of the disease and the milder the condition.

Therefore, the most fundamental and promising treatment strategy for SMA is to increase the expression level of the SMN protein. Currently, there are two main approaches for SMA treatment: one is to directly introduce a gene capable of encoding normal SMN1 via a vector to replace a defective SMN1 gene; the other is to modify the splicing of SMN2 mRNA to promote the normal expression of SMN2. Both methods aim to increase the expression level of the SMN protein. In addition, there are treatment strategies that do not rely on increasing SMN protein, such as stem cell therapy and neuromuscular protective drugs.

Over the past few decades, gene therapy based on adeno-associated virus (AAV) vectors has shown significant potential in the treatment of genetic diseases. The gene therapy method for SMA involves introducing an exogenous SMN1 gene via a viral vector to express normal SMN protein in the body, thereby treating the disease at its root cause. Compared with antisense nucleotide drugs or small-molecule drugs that require multiple administrations, gene therapy drugs have the advantage of providing long-term benefits with a single administration, making them more promising for application. However, issues such as the efficacy and safety of these SMA gene therapies need to be further optimized and addressed. For example, AAV vectors carrying the SMN1 gene have low expression efficiency and cannot effectively treat SMA; the human body has a T cell-mediated immune response to recombinant viral vectors, which may lead to liver damage and elevated transaminase levels; meanwhile, previous studies have found that when AAV vectors enter the central nervous system of nonhuman primates (NHPs) through the blood or cerebrospinal fluid, they can induce toxicity in the dorsal root ganglion (DRG), with the most severe toxicity manifested as ataxia. Traditional immunosuppressive regimens cannot prevent this toxicity, possibly because it may be caused by high transduction efficiency, which in turn induces cellular stress due to excessive transgene products in target cells.

Given the low I-year survival rate of Type 1 SMA patients both domestically and internationally, and the significant life threat faced by these patients, there is an urgent need to develop SMA therapeutic drugs that are long-lasting, effective, safe, and economically affordable to save the lives of SMA patients and improve their quality of life. Therefore, there is an urgent need to develop safer and more effective gene therapy drugs for the treatment of SMA.

It should be noted that the methods described in this section are not necessarily methods that have been previously conceived or employed. Unless otherwise specified, it shall not be assumed that any method described in this section is regarded as prior art merely by virtue of its inclusion in this section. Similarly, unless otherwise specified, the problems mentioned in this section should not be regarded as having been recognized in any prior art.

### SUMMARY

Based on this, the present application provides a gene expression cassette, wherein the gene expression cassette includes a polynucleotide encoding a human SMN protein, and the polynucleotide encoding the human SMN protein comprises a nucleotide sequence having at least 80%, 85%, 90%, 95%, 98%, 99%, or 100% identity to the nucleotide sequence shown in SEQ ID NO: 1 or SEQ ID NO: 3. The gene expression cassette in the present application improves the expression level of the SMN protein after SMN1 gene transduction by optimizing the codons of the SMN1 gene, so as to reduce the clinical dosage.

In some embodiments, a miRNA target sequence is also introduced into the gene expression cassette. By utilizing the characteristic of miRNA to inhibit gene expression at specific sites, the immune response of the body to AAV vectors and transgene products is alleviated, and the potential safety issues of gene therapy are reduced. In some preferred embodiments, the human miRNA target sequence includes at least one of a miR-183 target sequence, a miR-182 target sequence, a miR-96 target sequence, a miR23b target sequence, a miR-145 target sequence, a miR-148a target sequence, a miR-22 target sequence, a miR-122 target sequence, a miR-143 target sequence, a miR-21 target sequence, or a miR-192 target sequence. This is intended to reduce the expression of the SMN protein in the DRG and/or liver, thereby achieving the goal of alleviating DRG toxicity and liver toxicity caused by the AAV vectors.

According to one embodiment of the present application, a recombinant vector is further provided, wherein the recombinant vector comprises the gene expression cassette described in the present application. In some embodiments, the SMN1 gene expression cassette described in the present application is carried by an AAV vector, and the effects of different serotypes of AAV vectors, different doses of gene therapy drugs, and different administration routes on the therapeutic effect are compared, providing more options for solving issues such as the safety and efficacy of gene therapy.

According to one embodiment of the present application, a pharmaceutical composition is further provided, wherein the pharmaceutical composition includes the gene expression cassette described in the present application and the recombinant vector described in the present application.

According to one embodiment of the present application, an aqueous pharmaceutical formulation is further provided, wherein the aqueous pharmaceutical formulation includes the gene expression cassette described in the present application and the recombinant vector described in the present application.

According to one embodiment of the present application, a use of the gene expression cassette described in the present application, the recombinant vector described in the present application, the pharmaceutical composition described in the present application, or the aqueous pharmaceutical formulation described in the present application for expressing an SMN protein in cells is provided.

According to one embodiment of the present application, a use of the gene expression cassette described in the present application, the recombinant vector described in the present application, the pharmaceutical composition described in the present application, or the aqueous pharmaceutical formulation described in the present application in the preparation of a drug for preventing, alleviating, or treating a disease is further provided.

According to one embodiment of the present application, a method for preventing, alleviating, or treating a disease is further provided, the method includes administering the gene expression cassette described in the present application, the recombinant vector described in the present application, the pharmaceutical composition described in the present application, or the aqueous pharmaceutical formulation described in the present application to a subject in need thereof.

It should be understood that the content described in this section is not intended to identify key or important features of the embodiments of the present application, nor is it used to limit the scope of the present application. Other features of the present application will become easily understandable through the following specification.

### BRIEF DESCRIPTION OF THE DRAWINGS

The drawings exemplarily illustrate the embodiments and form part of the specification. Together with the textual description of the specification, they serve to explain the exemplary implementations of the embodiments. The shown embodiments are for illustrative purposes only and do not limit the scope of the claims. Throughout all the drawings, the same reference numerals refer to similar but not necessarily identical elements.
FIG. 1 is a schematic diagram of the structure of the scAAV-CMV/CBA-SMN1 vector prepared in Example 1.
FIG. 2 is a schematic diagram of the structure of the scAAV-CMV/CBA-coSMN1-1 vector prepared in Example 1.
FIG. 3 is a schematic diagram of the structure of the scAAV-CMV/CBA-coSMN1-2 vector prepared in Example 1.
FIG. 4 is a schematic diagram of the structure of the scAAV-CMV/CBA-coSMN1-2-TmiR vector prepared in Example 1.
FIG. 5 is a schematic diagram of the structure of the scAAV-CMV/CBA-coSMN1-3vector prepared in Example 1.
FIG. 6 is a schematic diagram of the structure of the scAAV-CMV/CBA-coSMN1-3-TmiR vector prepared in Example 1.
FIG. 7 is a graph of the Western blot results for characterizing SMN protein expression in Example 2. Among them, Lane 0 is a blank control group without any vector transfection; Lanes 1, 2, 3, 4, 5, and 6 are experimental groups transfected with scAAV-CMV/CBA-SMN1, scAAV CMV/CBA-coSMN1-1, scAAV-CMV/CBA-coSMN1-2, scAAV-CMV/CBA-coSMN1-2-TmiR, scAAV-CMV/CBA-coSMN1-3, and scAAV-CMV/CBA-coSMN1-3-TmiR plasmids, respectively.

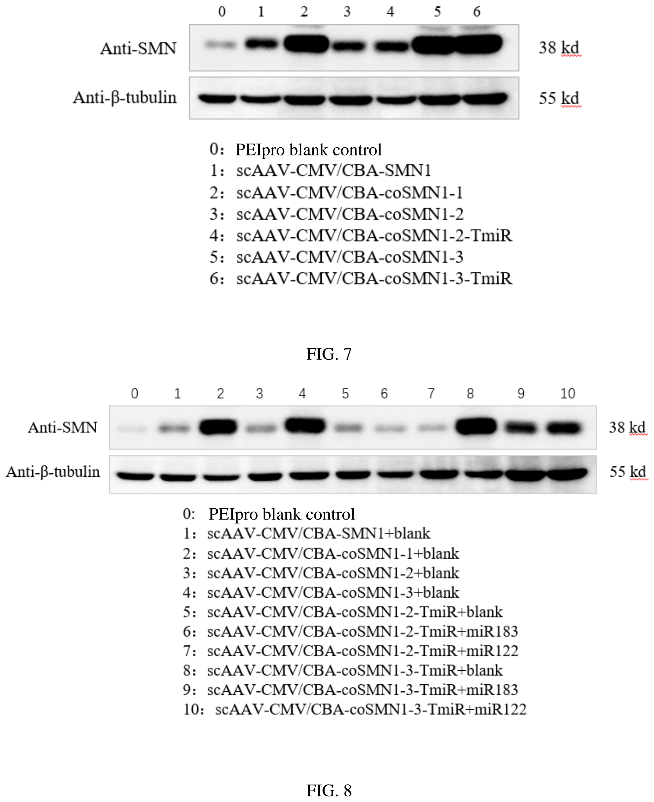

FIG. 8 is a graph of the Western blot results for characterizing SMN protein expression in Example 2. Among them, Lane 0 is a blank control group without any vector transfection; Lanes 1, 2, 3, 4, 5, and 8 are experimental groups transfected with scAAV-CMV/CBA-SMN1, scAAV-CMV/CBA-coSMN1-1, scAAV-CMV/CBA-coSMN1-2, scAAV-CMV/CBA-coSMN1-3, scAAV-CMV/CBA-coSMN1-2-TmiR, or scAAV-CMV/CBA-coSMN1-3-TmiR plasmids, respectively; Lanes 6 and 9 are experimental groups transfected with scAAV-CMV/CBA-coSMN1-2-TmiR or scAAV-CMV/CBA-coSMN1-3-TmiR plasmids, respectively, and also transfected with miR-183; and Lanes 7 and 10 are experimental groups transfected with scAAV-CMV/CBA-coSMN1-2-TmiR or scAAV-CMV/CBA-coSMN1-3-TmiR plasmids, respectively, and also transfected with miR-122.

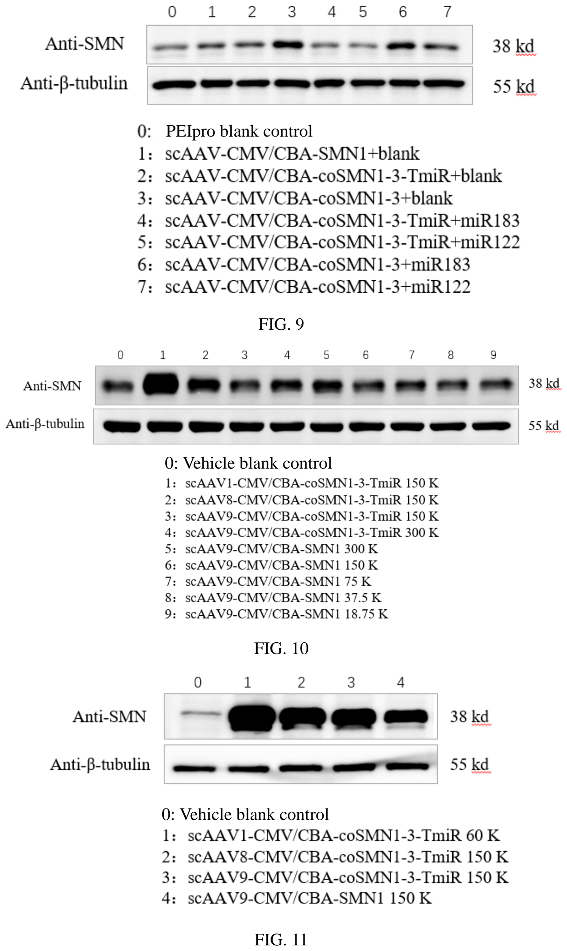

FIG. 9 is a graph of the Western blot results for characterizing SMN protein expression in Example 2. Among them, Lane 0 is a blank control group without any vector transfection; Lanes 1, 2, and 3 are experimental groups transfected with scAAV-CMV/CBA-SMN1, scAAV-CMV/CBA-coSMN1-3-TmiR, or scAAV-CMV/CBA-coSMN1-3 plasmids, respectively; Lanes 4 and 6 are experimental groups transfected with scAAV-CMV/CBA-coSMN1-3-TmiR or scAAV-CMV/CBA-coSMN1-3 plasmids, respectively, and also transfected with miR-183; and Lanes 5 and 7 are experimental groups transfected with scAAV-CMV/CBA-coSMN1-3-TmiR or scAAV-CMV/CBA-coSMN1-3 plasmids, respectively, and also transfected with miR-122.
FIG. 10 is a graph of the Western blot results for characterizing SMN protein expression in Example 3. Among them, Lane 0 is a blank control group without any vector transfection; Lanes 1, 2, 3, and 4 are experimental groups infected with viruses with different serotypes, i.e., scAAV1-CMV/CBA-coSMN1-3-TmiR, scAAV8-CMV/CBA-coSMN1-3-TmiR, scAAV9-CMV/CBA-coSMN1-3-TmiR, and scAAV9-CMV/CBA-coSMN1-3-TmiR, and experiments are conducted according to a multiplicity of infection of 150K, 150K, 150K, and 300K, respectively; and Lanes 5, 6, 7, 8, and 9 are experimental groups infected with virus scAAV9-CMV/CBA-SMN1 at a multiplicity of infection of 300K, 150K, 75K, 37.5K, and 18.75K, respectively.
FIG. 11 is a graph of the Western blot results for characterizing SMN protein expression in Example 3. Among them, Lane 0 is a blank control group without any vector transfection; Lanes 1, 2, 3, and 4 are experimental groups infected with viruses with different serotypes, i.e., scAAV1-CMV/CBA-coSMN1-3-TmiR, scAAV8-CMV/CBA-coSMN1-3-TmiR, scAAV9-CMV/CBA-coSMN1-3-TmiR, and scAAV9-CMV/CBA-SMN1, and experiments are conducted according to a multiplicity of infection of 60K, 150K, 150K, and 150K, respectively.
FIG. 12 is a line graph showing the survival cycle-survival rate of mice injected with different serotypes of recombinant viruses in Example 4.
FIG. 13 is a graph showing the body weight-survival time of mice injected with different serotypes of recombinant viruses in Example 4.
FIG. 14 is a graph showing the body length-survival time of mice injected with different serotypes of recombinant viruses in Example 4.
FIG. 15 is a graph showing the tail length-survival time of mice injected with different serotypes of recombinant viruses in Example 4.
FIG. 16 is a statistical graph of the righting reflex test results of mice injected with different serotypes of recombinant viruses in Example 4.
FIG. 17 is a statistical graph of the neurological scores of mice injected with different serotypes of recombinant viruses in Example 4.
FIG. 18 is a statistical graph of the rotarod test results of mice injected with different serotypes of recombinant viruses in Example 4.
FIG. 19 is a statistical graph of the experimental results of grip strength measurement of mice injected with different serotypes of recombinant viruses in Example 4.
FIG. 20 is a line graph showing the survival cycle-survival rate of mice injected with different doses of recombinant viruses in Example 4.
FIG. 21 is a graph showing the body weight-survival time of mice injected with different doses of recombinant viruses in Example 4.
FIG. 22 is a graph showing the body length-survival time of mice injected with different doses of recombinant viruses in Example 4.
FIG. 23 is a graph showing the tail length-survival time of mice injected with different doses of recombinant viruses in Example 4.
FIG. 24 is a statistical graph of the righting reflex test results of mice injected with different doses of recombinant viruses in Example 4.
FIG. 25 is a statistical graph of the neurological scores of mice injected with different doses of recombinant viruses in Example 4.
FIG. 26 is a statistical graph of the rotarod test results of mice injected with different doses of recombinant viruses in Example 4.
FIG. 27 is a statistical graph of the experimental results of grip strength measurement of mice injected with different doses of recombinant viruses in Example 4.
FIG. 28 is a statistical graph of the Western blot results of SMN protein expression in the brain, spinal cord, liver, and quadriceps tissues of mice in Example 4.
FIG. 29 is a statistical graph of the relative expression levels of SMN DNA content in the brain, spinal cord, DRG, and liver tissues of rhesus monkeys in Example 5.
FIG. 30 is a statistical graph of the relative expression levels of SMN mRNA content in the brain, spinal cord, DRG, and liver tissues of rhesus monkeys in Example 5.

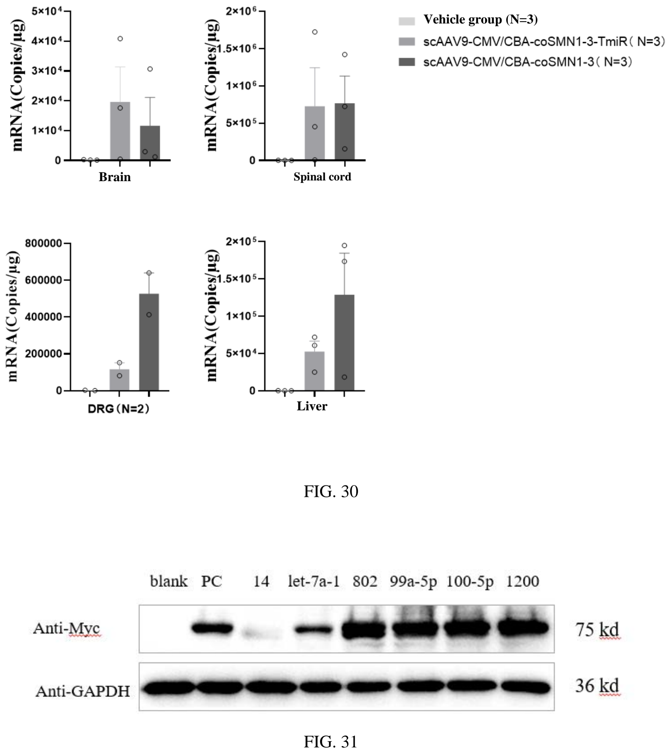

FIG. 31 shows a graph of the target protein expression results after transfection of Neuro-2a (N2a) cells with different plasmid vectors in Example 2.4. Among them, different labels in the figure represent transfection with different plasmid vectors: "blank" represents an untransfected blank group; "PC" represents a control vector without miR-183 and miR-122 target sequences; "14" represents a vector containing miR-183 and miR-122 target sequences; "Let-7a-1" represents a vector containing a let-7a-1 target sequence; "802" represents a vector containing a miR-802 target sequence; "99a-5p" represents a vector containing a miR-99a-5p target sequence; "100-5p" represents a vector containing a miR-100-5p target sequence; and "1200" represents a vector containing a miR-1200 target sequence.

### DETAILED DESCRIPTION OF EMBODIMENTS

Unless otherwise indicated, all numbers representing content, concentration, ratio, weight, particle diameter, percentage, technical effect, and so forth as used in the specification and claims are to be understood as being modified in any case by the term "about" or "approximately". Accordingly, unless indicated to the contrary, numerical parameters as set forth in the following specification and appended claims are approximations. Unless otherwise indicated, terms as used herein have the meanings commonly understood by those skilled in the art. For those skilled in the art, each numerical parameter may vary depending upon the desired properties and effects sought to be obtained by the present disclosure and should be construed in accordance with the number of significant figures and ordinary rounding techniques or in a manner understood by those skilled in the art.

Although the broad range of the numerical values and the parameters described in the present disclosure are approximate, the numerical values described in the specific embodiments are provided as accurately as possible. However, any numerical value inherently contains certain errors, which are inevitably caused by the standard deviation found in their respective test measurements. Each numerical range given in the specification will include every narrower numerical range that falls within such broader numerical range, as if such narrower numerical ranges were all explicitly written herein.

Unless otherwise indicated or contradictory to the context, the terms or expressions used herein should be read in conjunction with the entire content of the present disclosure and as understood by those skilled in the art. All technical and scientific terms used herein have the same meanings as commonly understood by those skilled in the art, unless otherwise defined.

As used herein, the expression "A and/or B" includes three cases: (1) A; (2) B; and (3) A and B. The expression "A, B and/or C" includes seven cases: (1) A; (2) B; (3) C; (4) A and B; (5) A and C; (6) B and C; and (7) A, B and C. The meaning of similar expressions may be deduced by analogy.

As used herein, "nucleic acid" and "polynucleotide" are used interchangeably and refer to a polymeric form of nucleotides of any length, including deoxyribonucleotides, ribonucleotides, combinations thereof, and analogs thereof.

As used herein, "polypeptide" and "peptide" are used interchangeably and refer to a polymer of amino acids of any length. Thus, polypeptides, oligopeptides, proteins, antibodies, and enzymes are all included within the definition of polypeptides.

It should be noted that in the context of the present application, "upstream" refers to the 5'-end of a gene or the N-terminus of a protein, and "downstream" refers to the 3'-end of a gene or the C-terminus of a protein. The direction from upstream to downstream is from the 5'-end to the 3'-end or from the N-terminus to the C-terminus.

As used herein, a "vector" refers to a nucleic acid molecule capable of transporting another nucleic acid molecule to which it is linked. Examples of vectors include, but are not limited to, plasmids, viruses, bacteria, bacteriophages, and insertable DNA fragments.

As used herein, an "adeno-associated viral vector" is derived from an adeno-associated virus. An "adeno-associated viral vector" is derived from the wild-type genome of the virus by using molecular methods to remove all or part of the wild-type genome and replace it with heterologous (non-native) nucleic acid (e.g., a nucleic acid encoding a therapeutic protein or polynucleotide sequence). Typically, for adeno-associated viral vectors, one or both inverted terminal repeats (ITRs) from the adeno-associated viral genome are retained in the adeno-associated viral vector. Adeno-associated viral vectors can be used as gene therapy vectors because they can introduce nucleic acid/genetic material into cells, allowing the nucleic acid/genetic material to remain in the cells.

As used herein, a "serotype" refers to an adeno-associated virus with a capsid that is serologically distinct from other adeno-associated viral serotypes. Serological specificity is determined based on the lack of cross-reactivity between antibodies against one adeno-associated virus compared with another adeno-associated virus. Such differences in cross-reactivity are typically caused by differences in capsid protein sequences/antigenic determinants (e.g., due to differences in the VP1, VP2, and/or VP3 sequences of the adeno-associated viral serotypes). Under the traditional definition, a serotype means that the virus of interest has been tested for neutralizing activity against sera specific for all existing and characterized serotypes, and no antibodies that neutralize the virus of interest have been found. As more naturally occurring viral isolates are discovered and/or capsid mutants are generated, there may or may not be serological differences from any of the currently existing serotypes. Therefore, in cases where a new virus (e.g., an adeno-associated virus) does not have serological differences, such a new virus (e.g., an adeno-associated virus) will be a subgroup or variant of the corresponding serotype. In many cases, serological testing for neutralizing activity has not been performed on mutant viruses with modified capsid sequences to determine whether they are another serotype under the traditional serotype definition. Therefore, for convenience and to avoid redundancy, the term "serotype" is used broadly to refer to serologically distinct viruses (e.g., adeno-associated viruses) as well as viruses (e.g., adeno-associated viruses) that may be within a subgroup or variant of a given serotype and are not serologically distinct.

As used herein, "miRNA target sequence" and "miRNA binding site" are used interchangeably and refer to a nucleotide sequence that can bind to a specific miRNA. In some non-limiting examples of the present application, the miRNA target sequence is designed at the 3' end of an SMN gene, and it can bind to a specific miRNA to inhibit the expression level of the SMN gene.

As used herein, "operably linked" refers to the linkage of multiple nucleic acid fragments in a functional relationship. A nucleic acid is "operably linked" to another nucleic acid sequence when it forms a functional relationship with the other nucleic acid sequence. For example, if a promoter or other transcriptional regulatory sequence affects the transcription of a coding sequence (or gene), it can be operationally linked to the coding sequence in a way that induces transcription of the gene. "Operably linked" means that the linked nucleotide sequences may be either contiguous or non-contiguous.

The terms "alleviation" and "treatment" and their synonyms as used herein refer to the amelioration of a disease, disorder and/or condition. "Alleviation" and "treatment" may be an improvement in at least one measurable physical parameter that is not necessarily identifiable by the patient. "Alleviation" and "treatment" may also be the physical (e.g., stabilization of recognizable symptoms), physiological (e.g., stabilization of physical parameters), or both inhibition of the development of the disease, disorder, and/or condition. "Alleviation" and "treatment" may also refer to slowing the progression of or reversal of a disease, disorder and/or condition.

As used herein, the term "prevention" and its synonyms refer to delaying the onset of specific diseases, disorders, and/or conditions or the symptoms associated with these diseases, disorders, and/or conditions, or reducing the risk of acquiring these diseases, disorders, and/or conditions.

To make the above objects, features, and advantages of the present application more clearly understandable, the specific embodiments of the present application are described in detail below.

### Gene Expression Cassette

According to one embodiment of the present application, a gene expression cassette is provided, wherein the gene expression cassette includes a polynucleotide encoding a human SMN protein, and the polynucleotide encoding the human SMN protein comprises a nucleotide sequence having at least 80%, 85%, 90%, 95%, 98%, 99%, or 100% identity to the nucleotide sequence shown in SEQ ID NO: 1 or SEQ ID NO: 3, such as 80%, 81%, 82%, 83%, 84%, 85%, 86%, 87%, 88%, 89%, 90%, 91%, 92%, 93%, 94%, 95%, 96%, 97%, 98%, 99%, 100%, etc., or other unlisted values within the range of 80% or more. In some embodiments, the polynucleotide encoding the human SMN protein comprises the nucleotide sequence shown in SEQ ID NO: 1 or SEQ ID NO: 3. In some preferred embodiments, the nucleotide sequence of the polynucleotide encoding the human SMN protein is selected from the nucleotide sequences shown in SEQ ID NO: 1 or SEQ ID NO: 3.

In some embodiments, the gene expression cassette further comprises a human miRNA target sequence. In some embodiments, the human miRNA target sequence is operably linked to the 3'-end of the polynucleotide encoding the human SMN protein. miRNAs (microRNAs) are single-stranded non-coding RNAs with a length of approximately 19 to 25 nucleotides, which are widely present in humans and animals. RNA-induced silencing complex formed by miRNAs and protein factors can recognize target sequences in mRNA and reduce the expression level of mRNA by degrading mRNA molecules, promoting 3'-end deadenylation of mRNA molecules, and inhibiting translation, thereby regulating gene expression at the post-transcriptional level (Kim VN. Nat Rev Mol Cell Biol. 2005; 6(5): 376-385.). By using a miRNA highly expressed in a certain type of cell and inserting the target sequence of the miRNA into the 3' UTR of an exogenous gene, the expression of the exogenous gene in this type of cell can be effectively inhibited. Therefore, by utilizing the characteristic of miRNA to inhibit gene expression, the immune response of the body to AAV vectors and transgene products is alleviated, and the potential safety issues of gene therapy are reduced.

In some embodiments, the human miRNA target sequence includes at least one of a miR-183 target sequence, a miR-182 target sequence, a miR-96 target sequence, a miR23b target sequence, a miR-145 target sequence, a miR-148a target sequence, a miR-22 target sequence, a miR-122 target sequence, a miR-143 target sequence, a miR-21 target sequence, or a miR-192 target sequence. In some preferred embodiments, the human miRNA target sequence includes at least one of a miR-183 target sequence or a miR-122 target sequence. In some more preferred embodiments, the human miRNA target sequence includes the nucleotide sequence shown in any one of SEQ ID NOs: 21-22. In some more preferred embodiments, the miR-183 target sequence includes the nucleotide sequence shown in SEQ ID NO: 21. In some more preferred embodiments, the miR-122 target sequence includes the nucleotide sequence shown in SEQ ID NO: 22. In some embodiments, the human miRNA target sequence can reduce the expression efficiency of the polynucleotide encoding the human SMN protein in liver tissue. In some embodiments, the human miRNA target sequence can reduce the expression efficiency of the polynucleotide encoding the human SMN protein in dorsal root ganglion tissue. The gene expression cassette provided by the present application carries one or more miRNA target sequences, which can effectively reduce the potential DRG toxicity and liver toxicity caused by AAV vectors carrying the SMN gene expression cassette.

In some embodiments, the number of the human miRNA target sequence is 1. In some embodiments, the number of the human miRNA target sequence is more than 1. In some embodiments, the more than 1 human miRNA target sequence is operably linked via at least one spacer sequence. In some embodiments, the spacer sequence comprises the nucleotide sequence shown in any one of SEQ ID NOs: 4-6. In some preferred embodiments, the human miRNA target sequence comprises 2 miR-183 target sequences and 2 miR-122 target sequences, and the 2 miR-183 target sequences and 2 miR-122 target sequences are sequentially linked via a spacer sequence shown in SEQ ID NO: 4, a spacer sequence shown in SEQ ID NO: 5, and a spacer sequence shown in SEQ ID NO: 6. In the gene expression cassette of the present application, an endogenous sequence shown in any one of SEQ ID NOs: 4-6 is selected as a spacer sequence to enhance the cooperative effect of different miRNAs.

In some embodiments, the gene expression cassette further includes a promoter. The promoter can be any suitable promoter sequence, i.e., a nucleic acid sequence recognizable by the host cell for expressing the nucleic acid sequence. The promoter sequence contains transcriptional regulatory sequences that mediate the expression of proteins or polypeptides. The promoter can be any nucleic acid sequence with transcriptional activity in the selected host cell, including mutated, truncated, and hybrid promoters, which can be derived from genes encoding extracellular or intracellular proteins or polypeptides that are homologous or heterologous to the host cell. In some non-limiting embodiments, the host cell is a nerve cell, and the promoter can be a nucleic acid sequence with transcriptional activity in nerve cells. In some embodiments, the promoter includes a CMV enhancer, a CBA promoter, a CAG promoter, a CBh promoter, an hSyn promoter, a CamKII promoter, an EF1α promoter, a UBC promoter, or an SMN endogenous promoter.

In some embodiments, the gene expression cassette further includes an enhancer. In some embodiments, the enhancer includes a human cytomegalovirus (CMV) enhancer.

In some embodiments, the gene expression cassette further includes a poly A sequence. Any transcription termination sequence known in the art can be used in the present application.

### Recombinant Vector, Pharmaceutical Composition, Aqueous Pharmaceutical Formulation

According to one embodiment of the present application, a recombinant vector is provided, wherein the recombinant vector comprises the gene expression cassette described in the present application. In some embodiments, the recombinant vector includes a recombinant plasmid expression vector or a recombinant viral vector. In some embodiments, the recombinant viral vector includes a recombinant adenoviral vector, a recombinant adeno-associated viral vector, a recombinant retroviral vector, a recombinant herpes simplex viral vector, or a recombinant vaccinia viral vector. In some embodiments, the recombinant viral vector is a recombinant adeno-associated viral vector. In some embodiments, the serotype of the recombinant adeno-associated viral vector includes AAV1, AAV2, AAV3, AAV4, AAV5, AAV6, AAV7, AAV8, AAV9, AAVrh10, AAV11, AAV 12, or AAV13. In some preferred embodiments, the serotype of the recombinant adeno-associated viral vector includes AAV1, AAV8, or AAV9. In some non-limiting embodiments, the recombinant viral vector is a serotype targeting nerve cells. The nucleotide sequences of the genomes of AAV serotypes are known in the art. In some embodiments, the gene expression cassette is located downstream of the 5' inverted terminal repeat sequence of the adeno-associated viral vector.

Viral particles comprising the gene expression cassette described in the present application are typically produced in packaging cells, which are capable of replicating the viral genome, expressing viral proteins, and assembling viral particles. Techniques for producing AAV vector particles in packaging cells are well-known in the art. In some non-limiting embodiments, packaging cells can be produced by simply transfecting suitable cells with one or more plasmids encoding the AAV genome, AAV proteins, and any required helper virus functions, known as the so-called "triple transfection" method: three plasmids are used, each carrying a set of such genes. See Grieger et al., Nature Protocols, 2006, 1(3): 1412-28.

According to one embodiment of the present application, a pharmaceutical composition is further provided, wherein the pharmaceutical composition includes the gene expression cassette described in the present application and the recombinant vector described in the present application. In some embodiments, the form of the pharmaceutical composition includes a lateral intraventricular injection drug, an intravenous injection drug, or an intrathecal injection drug. In some embodiments, in addition to the gene expression cassette and/or recombinant vector provided herein, the pharmaceutical composition of the present application may further comprise a pharmaceutically, nutritionally, or physiologically acceptable carrier, such as a liquid, gel, or solid carrier, an aqueous vehicle, a non-aqueous vehicle, an antimicrobial agent, an isotonic agent, a buffer, an antioxidant, a suspending agent, a dispersing agent, a chelating agent, a diluent, an adjuvant, an excipient, or a non-toxic auxiliary substance, as well as other components known in the art or various combinations thereof.

According to one embodiment of the present application, an aqueous pharmaceutical formulation is further provided, wherein the aqueous pharmaceutical formulation includes the gene expression cassette described in the present application or the recombinant vector described in the present application. In some embodiments, the aqueous pharmaceutical formulation includes a lateral intraventricular injection drug, an intravenous injection drug, or an intrathecal injection drug. In some embodiments, the aqueous pharmaceutical formulation further comprises one or more of Poloxamer 188, sodium chloride, potassium chloride, calcium chloride, magnesium chloride, and sodium phosphate buffer. In some embodiments, the aqueous pharmaceutical formulation further includes 0.001% Poloxamer 188, 150 mM sodium chloride, 3 mM potassium chloride, 1.4 mM calcium chloride, 0.8 mM magnesium chloride, and 1 mM sodium phosphate buffer. In some embodiments, the pH value of the aqueous pharmaceutical formulation is 6.4-7.6. In some embodiments, the pH value of the aqueous pharmaceutical formulation can be 6.4, 6.5, 6.6, 6.7, 6.8, 6.9, 7.0, 7.1, 7.2, 7.3, 7.4, 7.5, 7.6, or a value within a range formed by any two of the above points.

### Uses

According to one embodiment of the present application, a use of the gene expression cassette described in the present application, the recombinant vector described in the present application, the pharmaceutical composition described in the present application, or the aqueous pharmaceutical preparation described in the present application for expressing an SMN protein in cells is provided. The cells may be any cells of interest. In some embodiments, the cells are animal cells. In some embodiments, the animal cells include neuron.

According to one embodiment of the present application, a use of the gene expression cassette described in the present application, the recombinant vector described in the present application, the pharmaceutical composition described in the present application, or the aqueous pharmaceutical formulation described in the present application in the preparation of a drug for preventing, alleviating, or treating a disease is further provided. In some embodiments, the disease is an SMN-related disease. In some embodiments, the SMN-related diseases include: a disease related to insufficient SMN protein expression, a disease related to SMN protein deficiency, a disease related to SMN1 gene deletion, or a disease related to SMN1 gene mutation. In some preferred embodiments, the SMN-related disease is spinal muscular atrophy. In some embodiments, the spinal muscular atrophy includes SMA Type 1, SMA Type 2, SMA Type 3, or SMA Type 4. In some embodiments, the drug includes a lateral intraventricular injection drug, an intravenous injection drug, or an intrathecal injection drug.

### Disease Treatment Methods

According to one embodiment of the present application, a method for preventing, alleviating, or treating a disease is further provided, the method includes administering the gene expression cassette described in the present application, the recombinant vector described in the present application, the pharmaceutical composition described in the present application, or the aqueous pharmaceutical formulation described in the present application to a subject in need thereof. In some embodiments, the disease is an SMN-related disease. In some embodiments, the SMN-related diseases include: a disease related to insufficient SMN protein expression, a disease related to SMN protein deficiency, a disease related to SMN1 gene deletion, or a disease related to SMN1 gene mutation. In some preferred embodiments, the SMN-related disease is spinal muscular atrophy. In some embodiments, the spinal muscular atrophy includes SMA Type 1, SMA Type 2, SMA Type 3, or SMA Type 4.

The various embodiments and preferred options of the present application described above may be combined with each other (provided that they are not inherently contradictory to each other) and are applicable to the uses of the present application. All various embodiments formed by such combinations shall be deemed as part of the present application.

### Examples

The following provides an explanation of exemplary embodiments of the present application in conjunction with the accompanying drawings, including various details of the embodiments of the present application to aid understanding. It should be understood that these embodiments are merely exemplary and are in no way intended to limit the protection scope of the present application. The protection scope of the present application is only defined by the claims. Therefore, those skilled in the art should recognize that various changes and modifications can be made to the embodiments described herein without departing from the scope of the present application. Similarly, for clarity and conciseness, descriptions of well-known functions and structures are omitted in the following description.

Unless otherwise indicated, the reagents and instruments used in the following embodiments are all conventional products that are commercially available. Unless otherwise indicated, experiments are performed under conventional conditions or conditions recommended by the manufacturer.

### Example 1: Construction of Plasmid Vectors

### 1.1 Construction of scAAV-CMV/CBA-SMN1, scAAV-CMV/CBA-coSMN1-1, scAAV-CMV/CBA-coSMN1-2-TmiR, and scAAV-CMV/CBA-coSMN1-3-TmiR Plasmid Vectors

The mRNA sequence of a SMN1 gene (GenBank: NM_000344.4) was searched through the NCBI GenBank database (https://www.ncbi.nlm.nih.gov/gene). The coding region sequence of a SMN protein was analyzed from this mRNA sequence, and a SMN1 sequence (SEQ ID NO: 8) was obtained. Codon optimization was performed on the coding region sequence of the SMN protein to obtain coSMN1-1 (SEQ ID NO: 1), coSMN1-2 sequence (SEQ ID NO: 2), and coSMN1-3 sequence (SEQ ID NO: 3).

miR-183 (SEQ ID NO: 21) and miR-122 (SEQ ID NO: 22) target sequences were introduced at the 3' end of the coSMN1-2 and coSMN1-3 sequences, and each of the miR-183 and miR-122 target sequences was repeated once (SEQ ID NO: 7). The miR-183 and miR-122 target sequences were linked in series via spacer sequences (SEQ ID NO: 4, SEQ ID NO: 5, and SEQ ID NO: 6).

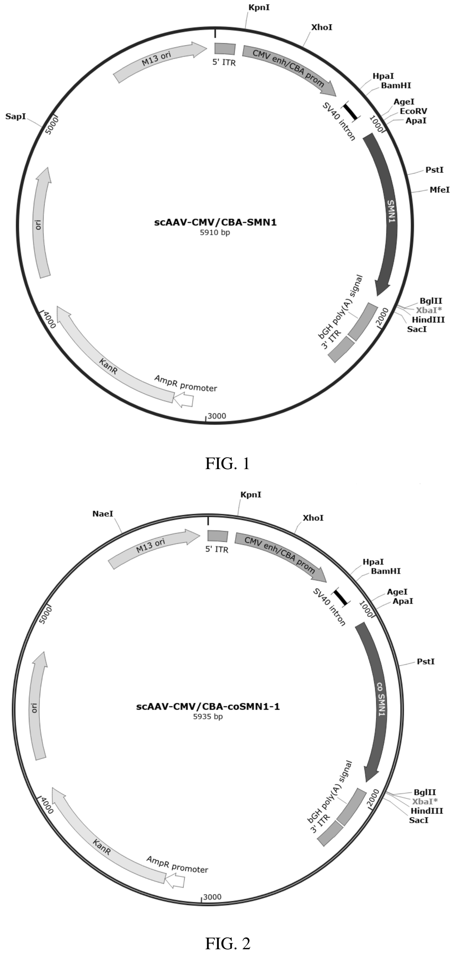

The sequences of scAAV-CMV/CBA-SMN1 (SEQ ID NO: 9), scAAV-CMV/CBA-coSMN1-1 (SEQ ID NO: 10), scAAV-CMV/CBA-coSMN1-2-TmiR (SEQ ID NO: 11), and scAAV-CMV/CBA-coSMN1-3-TmiR (SEQ ID NO: 12) were synthesized by Nanjing Genscript Biotechnology Co., Ltd., and the structures of the obtained vectors were as follows:
A schematic diagram of the structure of the scAAV-CMV/CBA-SMN1 plasmid vector was shown in FIG. 1. This plasmid comprises inverted terminal repeats (ITR), with the 5' end modified and the 3' end unmodified, and comprises a chicken β-actin promoter (CBA promoter), a human cytomegalovirus (CMV) enhancer, a SMN1 gene sequence (SEQ ID NO: 8), and a bovine growth hormone (BGH) polyadenylation signal (polyA).

A schematic diagram of the structure of the scAAV-CMV/CBA-coSMN1-1 plasmid vector was shown in FIG. 2. This plasmid comprises inverted terminal repeats, with the 5' end modified and the 3' end unmodified, and comprises a chicken β-actin promoter (CBA promoter), a human cytomegalovirus (CMV) enhancer, a coSMN1-1 (SEQ ID NO: 1), and a bovine growth hormone (BGH) polyadenylation signal (polyA).

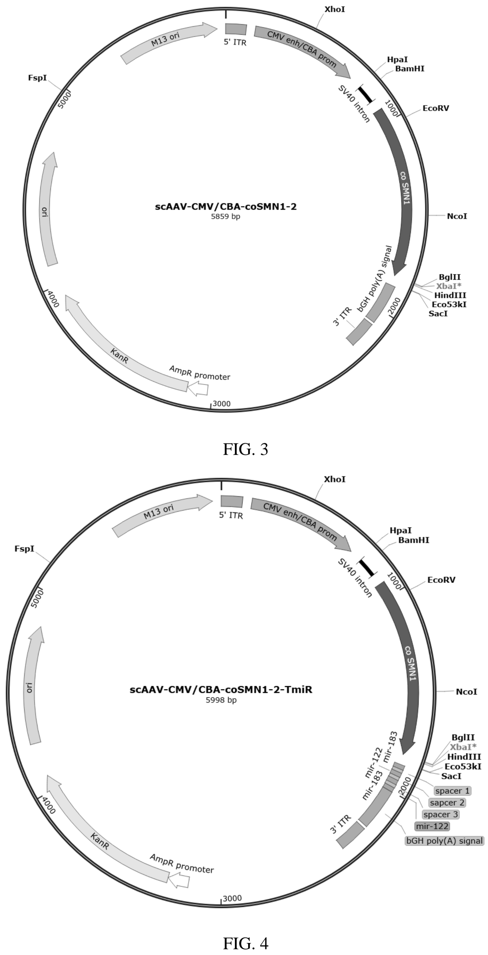

A schematic diagram of the structure of the scAAV-CMV/CBA-coSMN1-2-TmiR plasmid vector was shown in FIG. 4. This plasmid comprises inverted terminal repeats, with the 5' end modified and the 3' end unmodified, and comprises a chicken β-actin promoter (CBA promoter), a human cytomegalovirus (CMV) enhancer, coSMN1-2 (SEQ ID NO: 2), miR-183 and miR-122 target sequences (SEQ ID NO: 7), and a bovine growth hormone (BGH) polyadenylation signal (polyA).

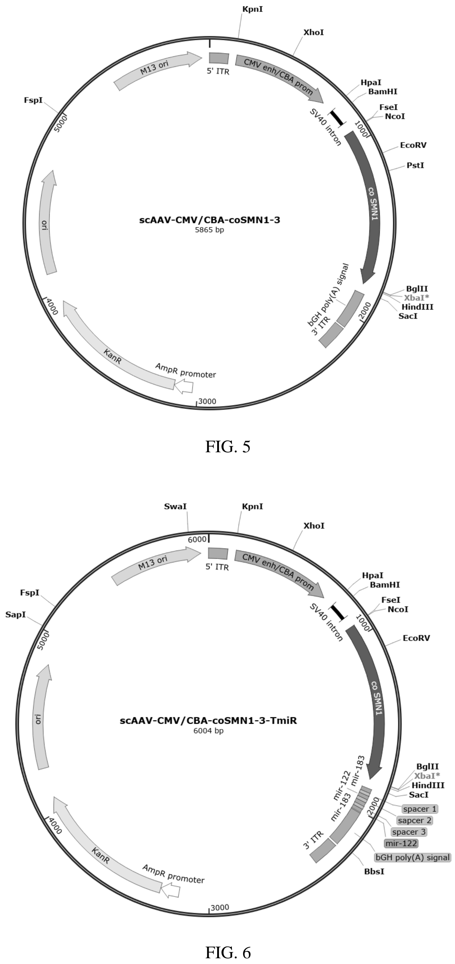

A schematic diagram of the structure of the scAAV-CMV/CBA-coSMN1-3-TmiR plasmid vector was shown in FIG. 6. This plasmid comprises inverted terminal repeats, with the 5' end modified and the 3' end unmodified, and comprises a chicken β-actin promoter (CBA promoter), a human cytomegalovirus (CMV) enhancer, coSMN1-3 (SEQ ID NO: 3), miR-183 and miR-122 target sequences (SEQ ID NO: 7), and a bovine growth hormone (BGH) polyadenylation signal (polyA).

### 1.2 Construction of scAAV-CMV/CBA-coSMN1-2 and scAAV-CMV/CBA-coSMN1-3 Plasmid Vectors

The following primers were synthesized by Tsingke Biotechnology Co., Ltd.: p101-line-F (SEQ ID NO: 15) and p101-line-R (SEQ ID NO: 16).

Using the scAAV-CMV/CBA-coSMN1-2-TmiR sequence and scAAV-CMV/CBA-coSMN1-3-TmiR synthesized in Example 1.1 as templates respectively, p101-line-F/R primers and KOD enzyme (#KOD-201B, TOYOBO) were added for PCR amplification of fragments. The PCR products were subjected to agarose gel electrophoresis, and the target bands were recovered. The recovered products were respectively ligated using Seamless Assembly (#C5891-50, Clone Smarter). The ligation products were transformed by stble3 competent cells (#CD521-02, Beijing TransGen Biotechnology Co., Ltd.), and the transformed products were spread on plates containing kanamycin, followed by incubation at 37°C overnight. Colonies were picked, and the bacterial solutions were sent to Tsingke Biotechnology for sequencing. Plasmids were extracted from colonies with correct sequences to obtain an scAAV-CMV/CBA-coSMN1-2 plasmid vector (SEQ ID NO: 13) and an **scAAV-CMV/CBA-coSMN1-3** plasmid vector (SEQ ID NO: 14). The structure of the obtained vectors was as follows:
A schematic diagram of the structure of the scAAV-CMV/CBA-coSMN1-2 plasmid vector was shown in FIG. 3. This plasmid comprises inverted terminal repeats, with the 5' end modified and the 3' end unmodified, and comprises a chicken β-actin promoter (CBA promoter), a human cytomegalovirus (CMV) enhancer, a coSMN1-2 (SEQ ID NO: 2), and a bovine growth hormone (BGH) polyadenylation signal (polyA).

A schematic diagram of the structure of the scAAV-CMV/CBA-coSMN1-3 plasmid vector was shown in FIG. 5. This plasmid comprises inverted terminal repeats, with the 5' end modified and the 3' end unmodified, and comprises a chicken β-actin promoter (CBA promoter), a human cytomegalovirus (CMV) enhancer, a coSMN1-3 (SEQ ID NO: 3), and a bovine growth hormone (BGH) polyadenylation signal (polyA).

### Example 2: Evaluation Experiment of In Vitro SMN Protein Expression Level by Plasmid Vectors

### 2.1 Evaluation of Codon Optimization Effect

### (1) Experimental Steps

SMN1 gene plasmid vectors without codon optimization (scAAV-CMV/CBA-SMN1) and SMN1 gene plasmid vectors with codon optimization (scAAV-CMV/CBA-coSMN1-1, scAAV-CMV/CBA-coSMN1-2, scAAV-CMV/CBA-coSMN1-2-TmiR, scAAV-CMV/CBA-coSMN1-3, and scAAV-CMV/CBA-coSMN1-3-TmiR) were respectively introduced into a HEK 293 cell line (purchased from ATCC) using PEIpro (#115-100, Polyplus) transfection reagent. The blank group without plasmid transfection was used as the negative control group. After 48 hours of incubation, the cells were collected to prepare protein samples, and Western blot was used to verify the SMN protein expression levels of the above plasmid vectors at the same dose.

The specific steps of Western blot were as follows: SDS-PAGE gels were prepared using the Epizyme PAGE gel rapid preparation kit (#PG112, Shanghai Epizyme Biomedical Technology Co., Ltd).A comb was removed, and the above protein samples were added to the comb for electrophoresis. When bromophenol blue electrophoresis reaches the position close to the bottom edge of a glass plate, electrophoresis was stopped, and membrane transfer was prepared. After membrane transfer was completed, the PVDF membrane (#10600023, Amersham) was taken out and rinsed once with 1×TBST for 3 minutes. The membrane was placed in a small box, and an appropriate amount of 5% non-fat milk was added (the liquid should submerge the membrane), followed by incubation on a shaker at room temperature for 1 hour. For primary antibody incubation, antibodies were prepared using 1×TBST. The blocked PVDF membrane was taken out, rinsed once with TBST, and then placed in an incubation box. Corresponding antibodies, including a SMN antibody (#60154-1-Ig, Proteintech) and a β-tubulin antibody (#66240-1-Ig, Proteintech), were added, and incubation was carried out at 4°C overnight. The antibodies were aspirated, and the PVDF membrane was rinsed three times with 1×TBST. For secondary antibody incubation, a mouse secondary antibody (#115-035-003, Jacksonimmuno) was prepared using 1×TBST, followed by incubation on a shaker at room temperature for 1 hour. The antibodies were aspirated, and PVDF was rinsed three times with 1×TBST. ECL (#SB-WB102, Shanghai Share-Bio Co., Ltd.) was used for development, and images were taken.

### (2) Experimental Results

The experimental results of Example 2.1 were shown in FIG. 7. The results in FIG. 7 indicated that compared with the blank control and the scAAV-CMV/CBA-SMN1 without codon optimization, the SMN protein expressions of the scAAV-CMV/CBA-coSMN1-1, scAAV-CMV/CBA-coSMN1-3, and scAAV-CMV/CBA-coSMN1-3-TmiR plasmid vectors were significantly increased, while the SMN protein expressions of the scAAV-CMV/CBA-coSMN1-2 and scAAV-CMV/CBA-coSMN1-2-TmiR did not show significant increases. The above results demonstrated that the codon-optimized coSMN1-1 and coSMN1-3 sequences significantly improved the SMN protein expression efficiency; however, the coSMN1-2 sequence, which also underwent codon optimization, did not achieve an increase in expression efficiency. In addition, besides coSMN1-2, the applicants also found a large number of optimized sequences that could not improve the expression efficiency during the screening process. This confirmed that the strategy of improving expression efficiency through codon optimization was affected by various factors such as protein type, protein length, secondary structure, and species type, and thus had unpredictability. Therefore, the preferred sequences in the vectors provided in the present application that could efficiently express SMN1 were coSMN1-1 and coSMN1-3.

### 2.2 Evaluation of Effect of Introducing miRNA Target Sequences

### (1) Experimental Steps

The experimental process was the same as that in Example 2.1, except that the experimental subjects were replaced as follows: scAAV-CMV/CBA-SMN1, scAAV-CMV/CBA-coSMN1-1, scAAV-CMV/CBA-coSMN1-2, scAAV-CMV/CBA-coSMN1-3, scAAV-CMV/CBA-coSMN1-2-TmiR, and scAAV-CMV/CBA-coSMN1-3-TmiR were respectively transfected into HEK 293 cells; scAAV-CMV/CBA-coSMN1-2-TmiR and scAAV-CMV/CBA-coSMN1-3-TmiR were respectively co-transfected with miR-183 into HEK 293 cells; and scAAV-CMV/CBA-coSMN1-2-TmiR and scAAV-CMV/CBA-coSMN1-3-TmiR were respectively co-transfected with miR-122 into HEK 293 cells. Similarly, the blank group without plasmid transfection was used as the negative control group.

### (2) Experimental Results

The experimental results of Example 2.2 were shown in FIG. 8. The results in FIG. 8 showed that when miRNAs (such as miR-183 or miR-122) were present in the cells, and the miRNA target sequences (specifically, the miR-183 target sequence and the miR-122 target sequence) were introduced into the **scAAV-CMV/CBA-coSMN1-3** plasmid vector at the same time, the SMN protein expression level in the cells could be significantly inhibited; however; however, when no miRNA target sequence was introduced into the plasmid vector, the SMN protein expression level in the cells could not be inhibited.

### 2.3 Evaluation of Effect of Introducing miRNA Target Sequences

### (1) Experimental Steps

The experimental process was the same as that in Example 2.1, except that the experimental subjects were replaced as follows: scAAV-CMV/CBA-SMN1, scAAV-CMV/CBA-coSMN1-3, and scAAV-CMV/CBA-coSMN1-3-TmiR were respectively transfected into Huh7 cells; **scAAV-CMV/CBA-coSMN1-3** and scAAV-CMV/CBA-coSMN1-3-TmiR were respectively co-transfected with miR-183 into Huh7 cells (donated by Shanghai Institute of Materia Medica); and scAAV-CMV/CBA-coSMN1-3 and scAAV-CMV/CBA-coSMN1-3-TmiR were respectively co-transfected with miR-122 into Huh7 cells. Similarly, the blank group without plasmid transfection was used as the negative control group.

### (2) Experimental Results

The experimental results of Example 2.3 were shown in FIG. 9. The results in FIG. 9 indicated that when miRNAs (such as miR-183 or miR-122) were present in the cells, comparisons between Band 3 and Band 4, Band 5, between Band 4 and Band 6, and between Band 5 and Band 7 showed that when the miRNA target sequences (specifically, the miR-183 target sequence and the miR-122 target sequence) were introduced into the scAAV-CMV/CBA-coSMN1-3-TmiR plasmid vector, the SMN protein expression level in the cells could be significantly inhibited; however, when no miRNA target sequence was introduced into the plasmid vector, the SMN protein expression level in the cells could not be inhibited.

### 2.4 Effect Comparison of Different miRNA Target Sequences

### (1) Experimental Steps

Control scAAV vectors comprising control miRNA target sequences were constructed according to the same method as in Example 1, and the construction method was briefly described as follows:
Amplification primers for control miRNAs (hsa-let-7a-1, hsa-miR-99a-5p, hsa-miR-100-5p, hsa-miR-802, hsa-miR-1200) were synthesized in the form of long primers. The primer sequences were shown in Table 1. Circular PCR was performed on the vector scAAV-MeP426-Mecp2-pA1-pA2 (SEQ ID NO: 38) and miRNA target sequences were introduced. The above amplified fragments were circularized via seamless cloning. The ligation products were transformed into recombinant enzyme-deficient Escherichia coli competent cells stabl3 (#CD521-01, Beijing TransGen Biotechnology Co., Ltd.) for amplification to obtain a variety of control scAAV vectors comprising control miRNA target sequences.

**Table 1 PCR Primer Sequences for Control miRNA**

| Name | Primer name | Primer sequence |
|---|---|---|
| hsa-let-7a-1 | let-7a-1-F | SEQ ID NO: 23 |
| | let-7a-1-R | SEQ ID NO: 24 |
| has-miR-99a-5p | 99a-5p-F | SEQ ID NO: 25 |
| | 99a-5p-R | SEQ ID NO: 26 |
| hsa-miR-100-5p | 100-5p-F | SEQ ID NO: 27 |
| | 100-5p-R | SEQ ID NO: 28 |
| hsa-miR-802 | 802-F | SEQ ID NO: 29 |
| | 802-R | SEQ ID NO: 30 |
| hsa-miR-1200 | 1200-F | SEQ ID NO: 31 |
| | 1200-R | SEQ ID NO: 32 |

After the construction was completed, according to the method in Example 2.1, the above-obtained control scAAV vectors comprising control miRNA target sequences were transfected into N2a cells, and then the expression level of the target protein was detected by Western blot. The results were shown in FIG. 31. Among them, different labels in the figure represented transfection with different vectors:
Blank represented an untransfected blank group,
"PC" refers to a control vector without miR-183 and miR-122 target sequences (scAAV-MeP426-Mecp2-pA1-pA2, and the sequence of the exogenous nucleic acid fragment between its ITR sequences was shown in SEQ ID NO: 38),
"14" refers to a vector containing miR-183 and miR-122 target sequences (scAAV-MeP426-Mecp2-pA1-pA2-183-483-122-130, and the sequence of the exogenous nucleic acid fragment between its ITR sequences was shown in SEQ ID NO: 39),
"Let-7a-1" refers to a vector containing a let-7a-1 target sequence (scAAV-MeP426-Mecp2-pA1-pA2-let-7a-1, and the sequence of the exogenous nucleic acid fragment between its ITR sequences was shown in SEQ ID NO: 33),
"802" refers to a vector comprising a miR-802 target sequence (scAAV-MeP426-Mecp2-pA1-pA2-802, and the sequence of the exogenous nucleic acid fragment between its ITR sequences was shown in SEQ ID NO: 36),
"99a-5p" refers to a vector comprising a miR-99a-5p target sequence (scAAV-MeP426-Mecp2-pA1-pA2-99a-5p, and the sequence of the exogenous nucleic acid fragment between its ITR sequences was shown in SEQ ID NO: 34),
"100-5p" refers to a vector comprising a miR-100-5p target sequence (scAAV-MeP426-Mecp2-pA1-pA2-100-5p, and the sequence of the exogenous nucleic acid fragment between its ITR sequences was shown in SEQ ID NO: 35), and
"1200" refers to a vector comprising a miR-1200 target sequence (scAAV-MeP426-Mecp2-pA1-pA2-1200, and the sequence of the exogenous nucleic acid fragment between its ITR sequences was shown in SEQ ID NO: 37).

### (2) Experimental Results

The results in FIG. 31 indicated that different combinations of the miRNA target sequences had different effects on reducing the expression of the target gene.

### Example 3: Preparation and In Vitro Efficacy Verification of AAV Recombinant Virus

### 3.1 Preparation of AAV Recombinant Virus

A three-plasmid packaging system was used to package a recombinant AAV virus, and affinity chromatography and anion exchange/multimodal chromatography were used for purification to obtain an AAV virus. AAV vector plasmids (scAAV-CMV/CBA-SMN1, scAAV-CMV/CBA-coSMN1-3-TmiR), AAV Rep and Cap protein expression plasmids (pAAV-R2C1, pAAV-R2C8, or pAAV-R2C9), and helper plasmid (pHelper) were mixed in an appropriate ratio, and then transfected into HEK293 cells using PEIPro. After 48 hours of transfection, the cells and culture supernatant were harvested. First, MaxNuclease benzonase nuclease (#NUC-SE101, Kactus Biosystems) was added to a final concentration of 100 U/mL, and then a HEPES (1% Tween 20, 2 mM MgCl₂) lysis buffer stock solution with a volume of 1/10 of the culture medium was added to a final concentration of 50 mM. The mixture was pipetted until the cells were completely suspended, then transferred to a shake flask, and shaken continuously at 37°C for 2 hours to fully degrade the nucleic acid. Then, 1/10 volume of 5 M NaCl and 10% sucrose solution was added, and the mixture was mixed well to obtain a lysed virus sample. The lysed virus sample was filtered using a Sartopure PP3 deep filter with a 0.45 µm pore size (#5051306P4, Sartorius). The filtered virus sample was concentrated and subjected to changing liquid using TFF. POROS^{™} CaptureSelect^{™} AAVX Affinity Resin (#A36740, Thermo Fisher) was used to capture the virus from the virus sample after changing liquid. For the AAV virus after affinity purification, Prima T (#311.5121-2, Sartorius) was used to remove empty capsid AAV virus from the virus. Finally, the recombinant viruses scAAV9-CMV/CBA-SMN1, scAAV1-CMV/CBA-coSMN1-3-TmiR, scAAV8-CMV/CBA-coSMN1-3-TmiR, and scAAV9-CMV/CBA-coSMN1-3-TmiR were obtained.

### 3.2 Detection of Genomic Copy Number

The ddPCR method was used to determine the genomic titer of the purified AAV virus. ddPCR primers and probes were designed for SMN1 and coSMN1-3:
SMN1-F: 5'-AGAGGAGCAAAATCTGTCCGA-3'
SMN1-probe: 5'-FAM-CCAGGAGACCTGGAGTTCTCAC-MGB-3'
SMN1-R: 5'-GGGTGGTGGAGGGAGAAAAG-3'
coSMN1-3-F: 5'-TACACAGGCTACGGCAACAG-3'
coSMN1-3-probe: 5'-FAM-ACCTCGCAAATTGGGCTCAGCA-MGB-3'
coSMN1-3-R: 5'-CTCGTTCTCTTGGGCGTTCT-3'

The primers and probes were synthesized by Genewiz Biotechnology Co., Ltd.

DNA samples, primers, probes, and ddPCR premix were mixed in proportions, added to a 96-well plate respectively, mixed well, and subjected to instantaneous centrifugation. After shaking and mixing thoroughly, centrifugation was performed at 2000 rpm for 1-2 minutes. An automatic droplet generator was turned on, a 96-well plate was placed inside, pipette tips were prepared, and the operation was started after confirmation to generate droplets. After droplet generation was completed, the newly generated 96-well plate containing oil and samples was taken out for droplet PCR amplification. After the PCR reaction was finished, the 96-well plate was removed and placed into a droplet reader, and relevant parameters were set: ddPCR supermix (no dUTP), FAM, etc. After the setup was completed, the droplet reading program was run. The reading unit was copies/µL. The copy number of the sample (copies/mL) was calculated according to the dilution factor.

### 3.3 In Vitro Efficacy Verification of AAV Recombinant Virus

### (1) Experimental Steps

scAAV9-CMV/CBA-SMN1, scAAV1-CMV/CBA-coSMN1-3-TmiR, scAAV8-CMV/CBA-coSMN1-3-TmiR, and scAAV9-CMV/CBA-coSMN1-3-TmiR recombinant virus were used to infect HEK293 cells and N2a cells.

After HEK293 cells were seeded in a 24-well plate, excess cells in two wells were taken for cell counting on the next day to calculate the average number of cells per well. Based on the number of cells, the scAAV9-CMV/CBA-SMN1 virus was diluted with complete culture medium to multiplicities of infection (MOI) of 300 K, 150 K, 75 K, 37.5 K, and 18.75 K; and the scAAV1-CMV/CBA-coSMN1-3-TmiR and scAAV8-CMV/CBA-coSMN1-3-TmiR viruses were diluted to an MOI of 150 K; the scAAV9-CMV/CBA-coSMN1-3-TmiR virus was diluted to MOIs of 300 K and 150 K respectively; and the cell culture medium in the well plate was aspirated, and a virus diluent was added to each well respectively. The blank group without virus addition was used as the negative control group. After 72 hours, the cells were collected to prepare protein samples, and Western blot as described in Example 2 was used to verify the expression of the SMN protein after the cells were infected with the virus.

After N2a cells were seeded in a 24-well plate, excess cells in two wells were taken for cell counting on the next day to calculate the average number of cells per well. Based on the number of cells, complete culture used to dilute scAAV9-CMV/CBA-SMN1, scAAV8-CMV/CBA-coSMN1-3-TmiR, and scAAV9-CMV/CBA-coSMN1-3-TmiR viruses with a multiplicity of infection (MOI) of 150 K using a complete culture medium; and the scAAV1-CMV/CBA-coSMN1-3-TmiR was diluted to an MOI of 60 K, and the cell culture medium in the well plate was aspirated, and a virus diluent was added to each well respectively. The blank group without plasmid transfection was used as the negative control group. After 72 hours, the cells were collected to prepare protein samples, and Western blot as described in Example 2 was used to verify the expression of the SMN protein after the cells were infected with the virus.

### (2) Experimental Results

The infection results of the HEK293 cells were shown in FIG. 10. Compared with the blank control and the scAAV9-CMV/CBA-SMN1 recombinant virus without codon optimization at a multiplicity of infection of 300 K, the scAAV1-CMV/CBA-coSMN1-3-TmiR and scAAV8-CMV/CBA-coSMN1-3-TmiR recombinant viruses significantly increased the expression of SMN protein at an MOI of 150 K. Compared with the negative control, the scAAV9-CMV/CBA-coSMN1-3-TmiR recombinant virus significantly increased the expression of the SMN protein at an MOI of 300 K. Among them, at the same MOI, the scAAV1-CMV/CBA-coSMN1-3-TmiR recombinant virus showed the highest SMN protein expression level.

The infection results of the N2a cells were shown in FIG. 11. Compared with the blank control and the scAAV9-CMV/CBA-SMN1 recombinant virus without codon optimization at the same multiplicity of infection, the scAAV1-CMV/CBA-coSMN1-3-TmiR, scAAV8-CMV/CBA-coSMN1-3-TmiR, and scAAV9-CMV/CBA-coSMN1-3-TmiR recombinant viruses all significantly increased the expression of the SMN protein after infecting the N2a cells.

In conclusion, infection with the scAAV1-CMV/CBA-coSMN1-3-TmiR, scAAV8-CMV/CBA-coSMN1-3-TmiR, and scAAV9-CMV/CBA-coSMN1-3-TmiR recombinant viruses of the present application could significantly increase the expression level of the SMN protein in vitro cells.

### Example 4: In Vivo Efficacy Evaluation Experiment of Recombinant AAV

FVB.cg-SMN2+/+; SMNA7+/+; Smn-/- (abbreviated as Smn+/-) mice were purchased from Jackson laboratory (#005025), and FVB.cg-SMN2+/+; SMNΔ7+/+; Smn-/- (abbreviated as Smn-/-) mice were obtained by breeding, which were used as an animal evaluation model for in vivo efficacy and safety of the recombinant virus. SMN genotype identification followed the instructions of Jackson laboratory, and the specific operation was as follows: PCR amplification was carried out using primers oIMR3439: TTTTCTCCCTCTTCAGAGTGAT (SEQ ID NO: 17); oIMR3440: CTGTTTCAAGGGAGTTGTGGC (SEQ ID NO: 18); oIMR7208: CTCCGGGATATTGGGATTG (SEQ ID NO: 19); and oIMR7210: GGTAACGCCAGGGTTTTCC (SEQ ID NO: 20); the amplified products were subjected to agarose electrophoresis, and mouse genotypes were identified according to the size of the electrophoresis bands: the wild-type band was 420 bp; the homozygous band was 500 bp; the heterozygous bands were two bands of 500 bp and 420 bp.

### 4.1 Efficacy Evaluation of Different Serotypes of AAV Recombinant Viruses on SMA Model Mice

### (1) Experimental Steps

scAAV9-CMV/CBA-SMN1 was used as a positive control, which was intravenously injected into 0-1-day-old Smn-/- mice at a dose of 5E+11 vg/mouse (virus genome, vg), with 9 mice were injected (calculated at 1.5 g per mouse). A vehicle group was used as a negative control (untreated group), and experimental groups were recombinant AAV viruses scAAV1-CMV/CBA-coSMN1-3-TmiR, scAAV8-CMV/CBA-coSMN1-3-TmiR, and scAAV9-CMV/CBA-coSMN1-3-TmiR. A microinjector (#10 µL, Shanghai Gaoge Industry and Trade Co., Ltd.) was used for a single bilateral intraventricular injection of 0-1-day-old Smn-/- mice at a dose of 5E+10 vg. Among them, 10 mice were injected in the negative control group, 13 mice were injected with scAAV1-CMV/CBA-coSMN1-3-TmiR, 10 mice were injected with scAAV8-CMV/CBA-coSMN1-3-TmiR, and 11 mice were injected with scAAV9-CMV/CBA-coSMN1-3-TmiR. After virus injection, the body weight, body length, and tail length of the mice were recorded daily (only information of surviving mice was recorded), and the survival rate and survival period of the mice were observed at the same time.

To observe the improvement effect of recombinant AAV with different serotypes on motor function, muscle weakness, and muscular atrophy of SMA model animals, the Righting reflex test was conducted daily on the 3rd day, 5th day, and from the 7th day to the 21st day after injection, and the time required for SMA model mice to right themselves from an abnormal posture to a normal posture was recorded; from the 21st day to the 63rd day, neurological score assessment was performed on the SMA model mice (for the scoring method, see Hatzipetros, et al. J. Vis. Exp. (104), e53257, doi:10.3791/53257 (2015)), and according to the scoring results, behavioral experiments were started on the SMA model mice on the 29th day, once a week. The behavioral experiments included: a rotarod test: a rotarod tester (#LE8205, Harvard Apparatus) was used to evaluate the motor coordination and balance ability of rodents, and the time required for the animals to fall off the rotarod tester was recorded; and grip strength measurement: a grip strength tester (#BIO-GS3, BIOSEB) was used to quantify the muscle grip strength of the animals.

### (2) Experimental Results

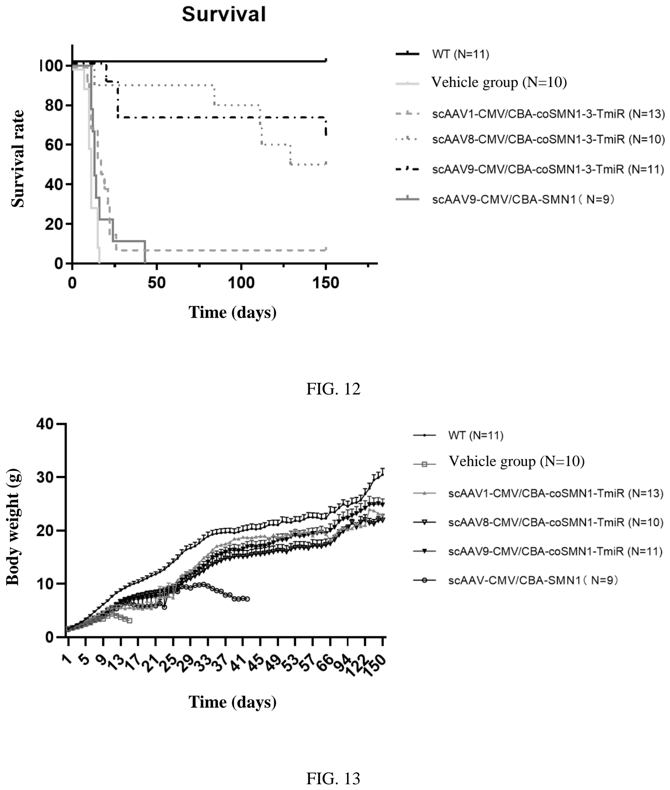

The results of survival rate and survival period were shown in FIG. 12. Compared with the negative control vehicle group and the positive control group (scAAV9-CMV/CBA-SMN1), injection of scAAV1-CMV/CBA-coSMN1-3-TmiR, scAAV8-CMV/CBA-coSMN1-3-TmiR, and scAAV9-CMV/CBA-coSMN1-3-TmiR recombinant viruses significantly prolonged the survival period of the SMA model mice and increased the survival rate of the mice. Among them, the SMA model mice injected with the scAAV8-CMV/CBA-coSMN1-3-TmiR and scAAV9-CMV/CBA-coSMN1-3-TmiR recombinant viruses showed a more significant increase in the survival rate.

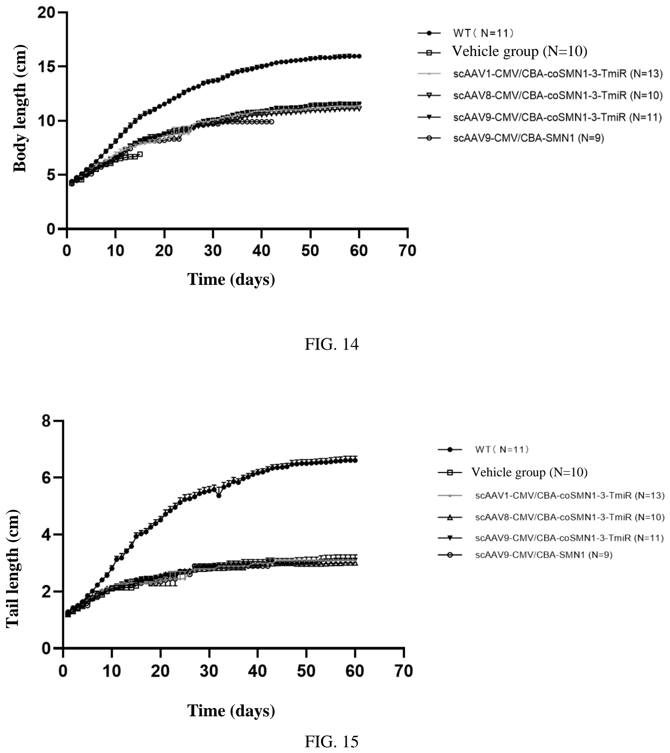

As could be seen from the results in FIGS. 13-15, compared with the negative control group and the positive control group, the body weight and body size of the SMA model mice injected with scAAV1-CMV/CBA-coSMN1-3-TmiR, scAAV8-CMV/CBA-coSMN1-3-TmiR, and scAAV9-CMV/CBA-coSMN1-3-TmiR recombinant viruses were restored to a certain extent, approaching the level of wild-type mice.

In conclusion, all three serotypes of the recombinant AAV viruses carrying the SMN gene with codon optimization and the miR-183 and miR-122 target sequences introduced into the SMN gene expression cassette could effectively restore the growth and development of the SMA model mice, increase their body weight, prolong their survival period, and improve their survival rate. Among them, the SMA model mice injected with the scAAV8-CMV/CBA-coSMN1-3-TmiR and scAAV9-CMV/CBA-coSMN1-3-TmiR recombinant viruses showed a more significant increase in the survival rate.

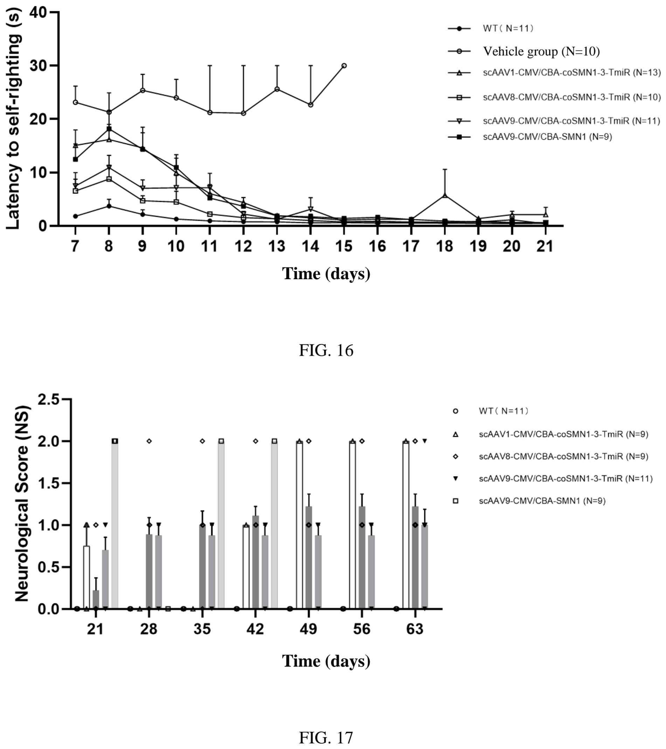

As shown in FIG. 16 (the righting reflex test results), with the extension of the survival period, the time required for the SMA model mice in the scAAV1-CMV/CBA-coSMN1-3-TmiR, scAAV8-CMV/CBA-coSMN1-3-TmiR, and scAAV9-CMV/CBA-coSMN1-3-TmiR recombinant virus injection groups to right themselves from an abnormal posture to a normal posture gradually shortened and restored to a level comparable to that of wild-type mice. This result indicated that different serotypes of the recombinant viruses in the present application could restore the motor behavior ability of the SMA model mice.

As shown in FIG. 17 (results of the neurological scores), before day 49, compared with the positive control group (scAAV9-CMV/CBA-SMN1), the SMA model mice in the recombinant virus injection groups (scAAV1-CMV/CBA-coSMN1-3-TmiR, scAAV8-CMV/CBA-coSMN1-3-TmiR, and scAAV9-CMV/CBA-coSMN1-3-TmiR) had lower neurological scores, indicating that the recombinant virus vectors provided in the present application could well restore the neurological function of the SMA model mice. After 49 days, almost all mice injected with the scAAV9-CMV/CBA-SMN1 recombinant virus in the positive control group died. Among the three experimental groups injected with different serotypes of the recombinant viruses, the scAAV9-CMV/CBA-coSMN1-3-TmiR recombinant virus injection group had the lowest score, followed by the scAAV8-CMV/CBA-coSMN1-3-TmiR recombinant virus injection group, and the scAAV1-CMV/CBA-coSMN1-3-TmiR recombinant virus injection group had a relatively higher score. The above experimental results indicated that the scAAV9-CMV/CBA-coSMN1-3-TmiR recombinant virus has the least impact on the abnormal state of the hind limbs of mice.

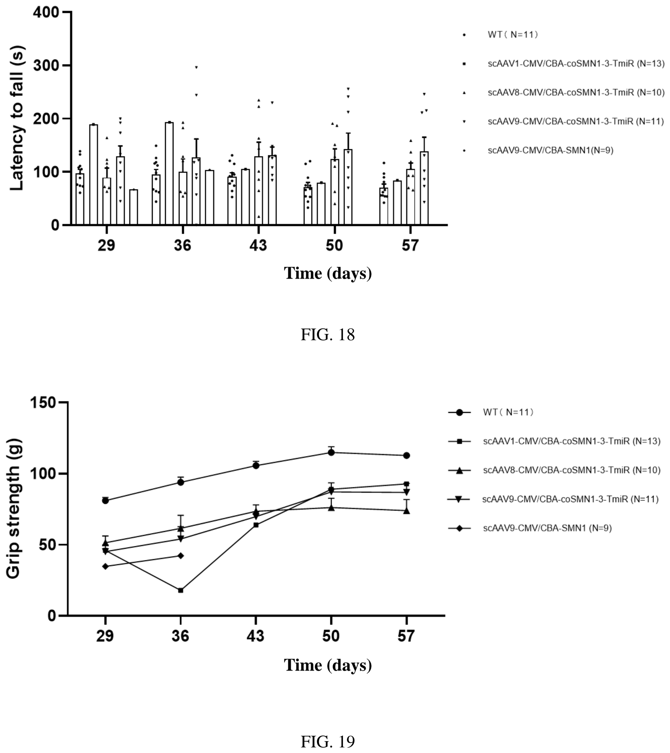

As shown in FIG. 18 (the rotarod test results), compared with the positive control group (scAAV9-CMV/CBA-SMN1), the mice in the recombinant virus injection groups (scAAV1-CMV/CBA-coSMN1-3-TmiR, scAAV8-CMV/CBA-coSMN1-3-TmiR, and scAAV9-CMV/CBA-coSMN1-3-TmiR) had a significantly longer duration on the rotarod. Compared with the time required for the wild-type mice to fall off the rotarod, the scAAV1-CMV/CBA-coSMN1-3-TmiR recombinant virus injection group had a significantly longer duration on the rotarod in the first two tests, and in the weekly tests during the subsequent survival period, the time required for the mice to fall off the rotarod was comparable to that of the wild-type mice, with no significant difference; the scAAV8-CMV/CBA-coSMN1-3-TmiR recombinant virus injection group had no significant difference in the duration on the rotarod compared with the wild-type mice in the first two tests, however, in the weekly tests during the subsequent survival period, the time required for the mice to fall off the rotarod was significantly longer than that of the wild-type mice, and the delay time continued to increase; and for the scAAV9-CMV/CBA-coSMN1-3-TmiR recombinant virus injection group, the time required for the mice to fall off the rotarod was significantly longer than that of the wild-type mice, and the difference became larger with the extension of the survival period. The above results indicated that all three serotypes of the recombinant AAV of the present application carrying the SMN gene with codon optimization and the miR-183 and miR-122 target sequences introduced into the SMN gene expression cassette could effectively restore the motor coordination ability of the SMA model mice; and the scAAV8-CMV/CBA-coSMN1-3-TmiR and scAAV9-CMV/CBA-coSMN1-3-TmiR recombinant viruses had better effects than the untreated wild-type mice.

As shown in FIG. 19 (results of grip strength measurement), with the extension of the survival period, the grip strength of the SMA model mice in the scAAV1-CMV/CBA-coSMN1-3-TmiR, scAAV8-CMV/CBA-coSMN1-3-TmiR, and scAAV9-CMV/CBA-coSMN1-3-TmiR recombinant virus injection groups continued to increase. After about 50 days of administration, the grip strength had restored to a level similar to that of the wild-type mice, with no significant difference among the three serotypes. The above results indicated that all three serotypes of the recombinant AAV viruses of the present application carrying the SMN gene with codon optimization and the miR-183 and miR-122 target sequences introduced into the SMN gene expression cassette could effectively reverse the muscle weakness state of the SMA model mice.

In conclusion, all three serotypes of the recombinant AAV viruses of the present application with the SMN gene with codon optimization and the miR-183 and miR-122 target sequences introduced into the SMN gene expression cassette were injected into the central nervous system via lateral intraventricular injection, which restored the growth and development of the SMA model mice, increased their body weight, body length, and tail length, prolonged the survival period of the SMA model animals, and improved their survival rate. At the same time, they also restored the motor coordination ability of the SMA model mice and reversed their states of muscle weakness and muscular atrophy. Among them, scAAV9-CMV/CBA-coSMN1-3-TmiR showed the best effect.

### 4.2 Efficacy Evaluation of Recombinant AAV with Different Doses on SMA Model Mice

### (1) Experimental Steps

For the scAAV9-CMV/CBA-coSMN1-3-TmiR recombinant virus, three different administration doses (high, medium, and low doses) were designed, which were 5E+10 vg/mouse, 2.5E+10 vg/mouse, and 1E+10 vg/mouse respectively. The recombinant virus was administered to 0-1-day-old Smn-/- mice via a single bilateral intraventricular injection, with 6-8 Smn-/- mice in each group. After the injection, the body weight, body length, and tail length of the mice were recorded daily (only data of surviving mice were recorded), and the survival period of the mice was observed. To observe the improvement effect of the recombinant vector on motor coordination, muscle weakness, and muscular atrophy in the SMA model animals, the Righting reflex test was conducted daily on the 3rd day, 5th day, and from the 7th day to the 21st day after virus injection, and the time required for SMA model mice to right themselves from an abnormal posture to a normal posture was recorded; from the 21st day to the 63rd day, neurological score assessment was performed on the SMA model mice, and according to the scoring results, behavioral experiments were started on the SMA model mice on the 29th day, once a week. The behavioral experiments included: a rotarod test: a rotarod tester was used to evaluate the motor coordination and balance ability of rodents, and the time required for the animals to fall off the rotarod tester was recorded; and grip strength measurement: a grip strength tester was used to quantify the muscle grip strength of the animals.

### (2) Experimental Results

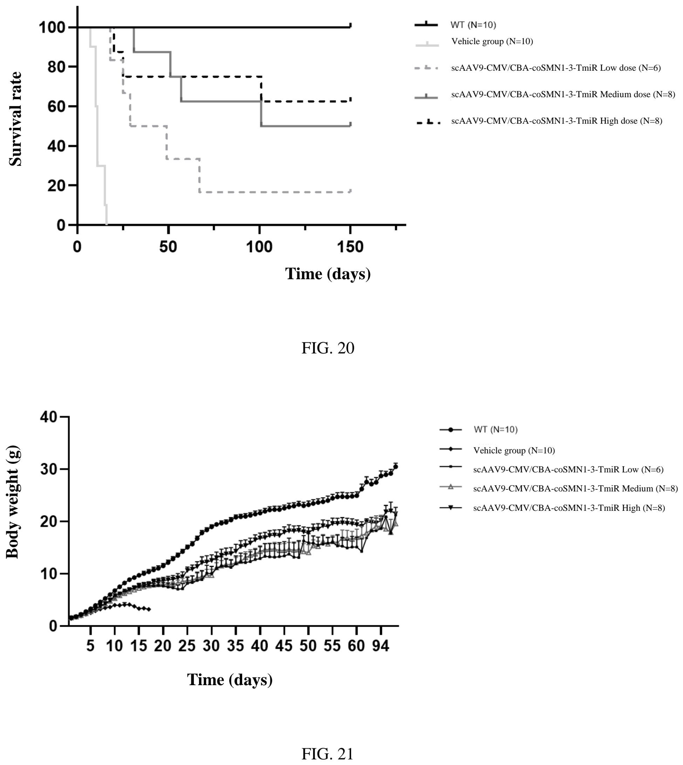

As shown in FIG. 20 (results of survival rate and survival period), compared with the SMA model mice not injected with the recombinant virus, all three doses of the scAAV9-CMV/CBA-coSMN1-3-TmiR recombinant virus could prolong the survival period of the SMA model mice. However, there were differences in the extent of survival period prolongation among different administration doses. The low-dose effect is weaker, while the medium dose and high-dose effects are comparable, both effectively prolonging the survival cycle of the SMA model mice.

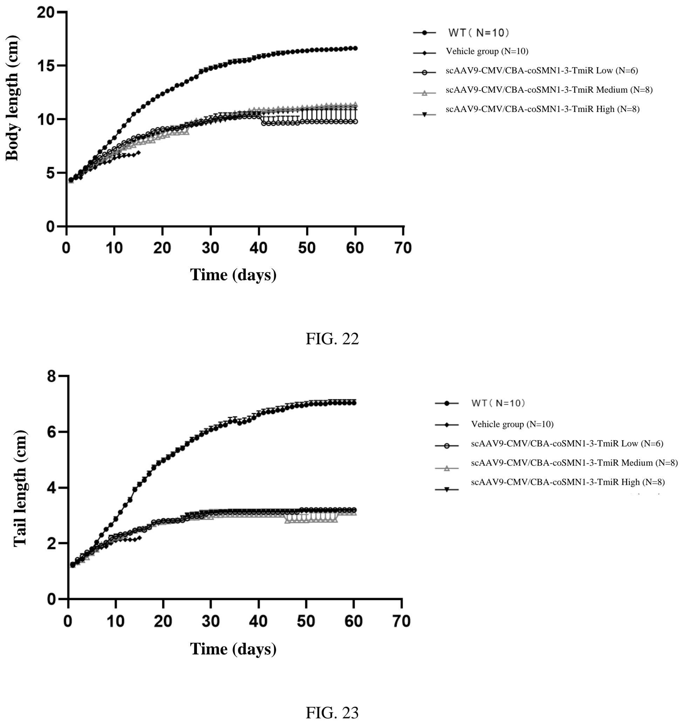

As shown in FIG. 21 (body weight), FIG. 22 (body length), and FIG. 23 (tail length), after injection of the three different doses of the scAAV9-CMV/CBA-coSMN1-3-TmiR recombinant virus, the body weight and body size of the mice gradually increased over time. Among them, the mice administered the low dose of scAAV9-CMV/CBA-coSMN1-3-TmiR showed a slower increase in body weight. The medium-dose and high-dose administration of scAAV9-CMV/CBA-coSMN1-3-TmiR was more effective in restoring the growth and development of the SMA model mice.

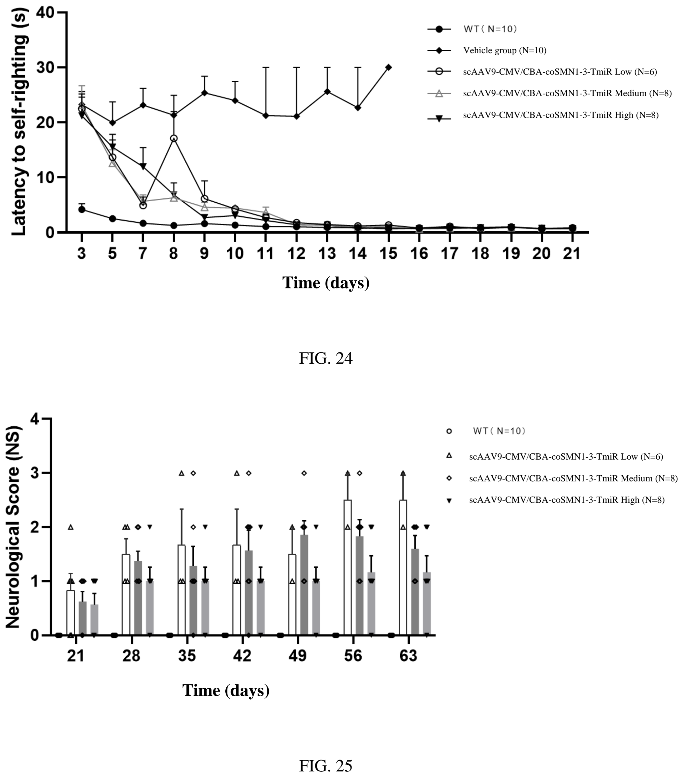

As shown in FIG. 24 (the righting reflex test results), with the extension of the survival period, the time required for the SMA model mice in the injection groups of three doses of scAAV9-CMV/CBA-coSMN1-3-TmiR to right themselves from an abnormal posture to a normal posture gradually shortened, and restored to a level comparable to that of the wild-type mice 12 days after administration. The above results indicated that all three doses of the scAAV9-CMV/CBA-coSMN1-3-TmiR recombinant virus could restore the motor behavior ability of the SMA model mice.

As shown in FIG. 25 (results of the neurological scores), the neurological scores of the SMA model mice in the injection groups of three doses of scAAV9-CMV/CBA-coSMN1-3-TmiR were slightly higher than those of the wild-type mice, indicating varying degrees of impact on the hind limb status of the mice.

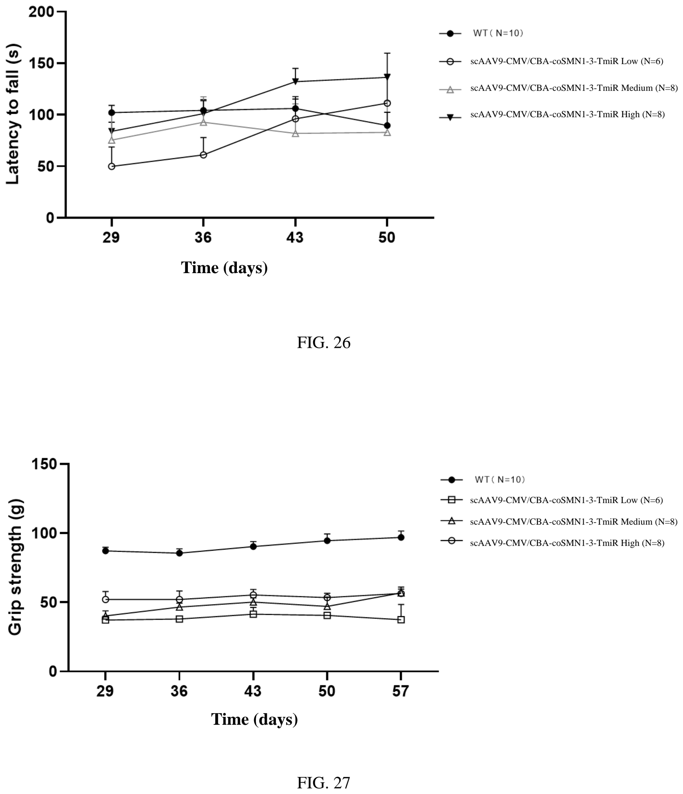

As shown in FIG. 26 (the rotarod test results), compared with the time required for the wild-type mice to fall off the rotarod, the medium-dose and high-dose virus injection groups of scAAV9-CMV/CBA-coSMN1-3-TmiR restored the duration on the rotarod to a level similar to that of the wild-type mice. The above results indicated that the medium and high doses of the scAAV9-CMV/CBA-coSMN1-3-TmiR recombinant virus were more effective in restoring the motor coordination ability of the SMA model mice.

As shown in FIG. 27 (results of grip strength measurement), with the extension of the survival period, the grip weight of the SMA model mice in the medium-dose and high-dose virus injection groups of scAAV9-CMV/CBA-coSMN1-3-TmiR continued to increase, and there was no significant difference between the medium and high doses. The above results indicated that both the medium-dose and high-dose administration of scAAV9-CMV/CBA-coSMN1-3-TmiR could effectively reverse the muscle weakness state of the SMA model mice.

In conclusion, after lateral intraventricular injection of low, medium, and high doses of the scAAV9-CMV/CBA-coSMN1-3-TmiR recombinant virus into the SMA model mice, all doses could restore the growth and development of the SMA model mice, increase their body weight, body length, and tail length, prolonged the survival period of SMA model animals, restore their motor coordination ability, and reverse their states of muscle weakness and muscular atrophy. Among them, the administration of medium and high doses showed better effects.

### 4.3 Effect of Recombinant AAV on SMN Protein Expression in Different Tissues of SMA Model Mice

### (1) Experimental Steps

The scAAV9-CMV/CBA-SMN1 and scAAV9-CMV/CBA-coSMN1-3-TmiR recombinant AAV viruses were administered to 0-1-day-old Smn-/- mice via a single bilateral intraventricular injection at a dose of 2.5E+10 vg using a microinjector, with 3 mice injected in each group. Mice in the untreated group were given a vehicle as a negative control. 7 days later, brain, spinal cord, liver, and quadriceps tissues were collected from mice in a wild-type vehicle group, scAAV9-CMV/CBA-SMN1 group, and scAAV9-CMV/CBA-coSMN1-3-TmiR group respectively.

An appropriate amount of tissue sample was taken, added with Ripa lysis buffer (#P0013B, Beyotime Biotechnology), and ground using a grinder (#scientz-48, Ningbo Scientz Biotechnology Co., Ltd.). Protein samples were prepared. Western blot detection was performed on protein samples from different tissues following the steps described in Example 2 to determine the SMN protein expression level in different tissues after virus injection.

### (2) Experimental Results

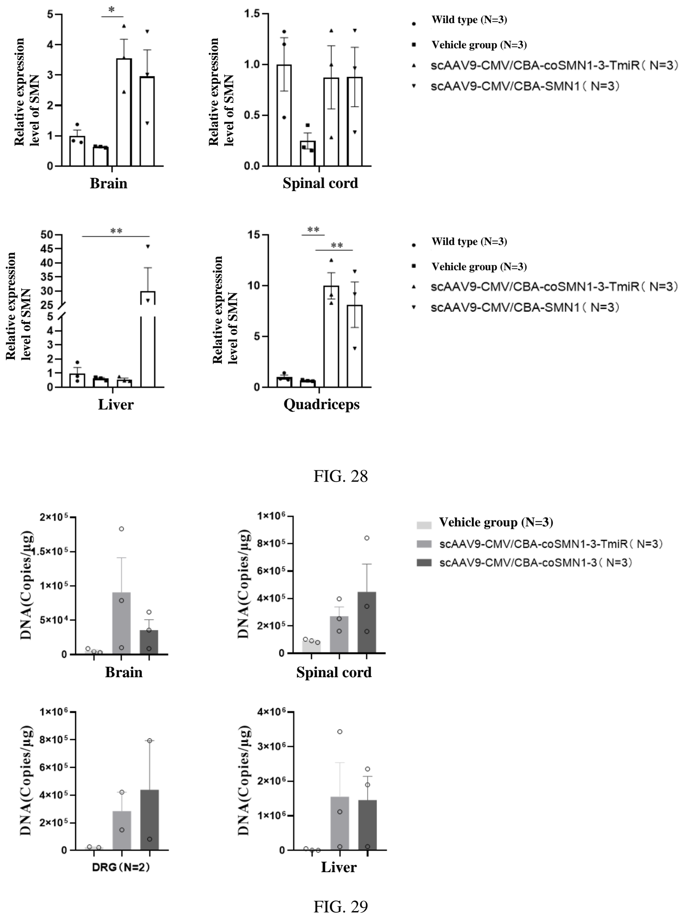

As shown in FIG. 28, compared with the untreated group, the scAAV9-CMV/CBA-SMN1 and scAAV9-CMV/CBA-coSMN1-3-TmiR injection groups showed significantly increased SMN protein expression levels in the brain, spinal cord, and quadriceps tissues, which were restored to or exceeded the SMN expression level of the wild-type mice. However, in the liver, the SMN protein expression level in the scAAV9-CMV/CBA-SMN1 group was significantly higher than that in the scAAV9-CMV/CBA-coSMN1-3-TmiR group and the wild-type mouse group. This suggested that the scAAV9-CMV/CBA-coSMN1-3-TmiR virus could targetedly reduce the SMN protein expression level in the liver, i.e., adding the miRNA target sequences to the viral vector could overcome the overexpression of the SMN protein in the liver, thereby avoiding potential hepatotoxicity caused by AAV therapy.

### Example 5: Evaluation of Biodistribution of Recombinant AAV after Single Intrathecal Injection in Juvenile Rhesus Monkeys

### (1) Experimental Steps

Juvenile rhesus monkeys were given a single intrathecal injection of a vehicle, scAAV9-CMV/CBA-coSMN1-3 (3E+13 vg/monkey), and scAAV9-CMV/CBA-coSMN1-3-TmiR (3E+13 vg/monkey), with 3 males in each group, and observed for 13 weeks. Terminal dissection was performed on Day 92, and brain, spinal cord, DRG, and liver tissues were collected from rhesus monkeys in the vehicle group, scAAV9-CMV/CBA-SMN1 group, and scAAV9-CMV/CBA-coSMN1-3-TmiR group (only two DRG tissue samples were collected).

Tissue DNA extraction: an appropriate amount of tissue sample was placed into a 1.5 ml EP tube, and tissue DNA was extracted using the DNeasy Blood & Tissue Kit (#69506, Qiagen). The DNA concentration was measured using a Nanodrop instrument (#nanodrop one, Thermo Fisher).

Tissue RNA extraction: an appropriate amount of tissue sample was placed into a 1.5 ml EP tube, added with Trizol (#15596018, Thermo Fisher) for RNA extraction, and the RNA was reverse-transcribed into cDNA.

The qPCR method was used to determine the copy number of tissue DNA and cDNA samples. The qPCR primers and probes were as follows:
coSMN1-3-F: 5'-TACACAGGCTACGGCAACAG-3'
coSMN1-3-probe: 5'-FAM-ACCTCGCAAATTGGGCTCAGCA-MGB-3'
coSMN1-3-R: 5'-CTCGTTCTCTTGGGCGTTCT-3'

The primers and probes were synthesized by Sangon Biotech (Shanghai) Co., Ltd.

Standards or DNA/cDNA samples, primers, probes, ROX Reference Dye II (#AM41818A, TaKaRa), and Premix Ex Taq^{™} (#RR390A, TaKaRa) were mixed in proportions to prepare a mixture, which were added to a 96-well plate respectively, mixed well, and subjected to instantaneous centrifugation. Each sample was set with 3 replicate wells. After shaking and mixing thoroughly, centrifugation was performed at 2000 rpm for 1-2 minutes. The 96-well plate was placed into a qPCR instrument (#QuantStudio5, Thermo Fisher) for PCR amplification. The copy number of the sample (copies/µg) was calculated based on the standard curve and dilution factor.

### (2) Experimental Results

As shown in FIG. 29, compared with the vehicle group, the scAAV9-CMV/CBA-coSMN1-3 and scAAV9-CMV/CBA-coSMN1-3-TmiR injection groups showed a tendency of increased SMN DNA content levels in the brain, spinal cord, DRG, and liver tissues compared with wild-type rhesus monkeys. This result indicated that intrathecal injection of the viral vector could deliver the target gene to tissues of the central nervous system and peripheral system.

As shown in FIG. 30, compared with the vehicle group, the scAAV9-CMV/CBA-coSMN1-3 and scAAV9-CMV/CBA-coSMN1-3-TmiR injection groups showed a tendency of increased SMN mRNA content levels in the brain, spinal cord, DRG, and liver tissues compared with wild-type rhesus monkeys. Among them, there was no significant difference in SMN mRNA content in the brain and spinal cord tissues between the scAAV9-CMV/CBA-coSMN1-3 and scAAV9-CMV/CBA-coSMN1-3-TmiR injection groups. However, in the DRG and liver tissues, the SMN mRNA content level in the scAAV9-CMV/CBA-coSMN1-3 group tended to be higher than that in the scAAV9-CMV/CBA-coSMN1-3-TmiR group. This suggested that the scAAV9-CMV/CBA-coSMN1-3-TmiR virus can targetedly reduce the SMN mRNA expression level in the liver and DRG, while not affecting the SMN mRNA expression level in the brain and spinal cord tissues. The above results indicated that adding the miRNA target sequences to the viral vector could overcome the overexpression of the SMN gene in the liver and DRG, thereby avoiding hepatotoxicity and DRG toxicity caused by AAV therapy.

It should be noted that the above are only preferred embodiments of the present application and are not intended to limit the present application. For those skilled in the art, various modifications and changes may be made to the present application. Although specific embodiments have been described, alternatives, modifications, changes, improvements, and substantial equivalents of the above embodiments may exist or be unforeseeable at present for the applicant or other skilled in the art. Therefore, the appended claims submitted and any claims that may be modified are intended to cover all such alternatives, modifications, changes, improvements, and substantial equivalents. Importantly, with the evolution of technology, many elements described herein may be replaced by equivalent elements that appear after the present application.

## Claims

1. A gene expression cassette, wherein the gene expression cassette includes a polynucleotide encoding a human SMN protein, wherein the polynucleotide encoding the human SMN protein comprises a nucleotide sequence having at least 80%, 85%, 90%, 95%, 98%, 99%, or 100% identity to the nucleotide sequence shown in SEQ ID NO: 1 or SEQ ID NO: 3.

2. The gene expression cassette according to claim 1, wherein the polynucleotide encoding the human SMN protein comprises the nucleotide sequence shown in SEQ ID NO: 1 or SEQ ID NO: 3.

3. The gene expression cassette according to claim 1, wherein the gene expression cassette further includes a human miRNA target sequence.

4. The gene expression cassette according to claim 3, wherein the human miRNA target sequence is operably linked to the 3'-end of the polynucleotide encoding the human SMN protein.

5. The gene expression cassette according to claim 3, wherein:
the human miRNA target sequence can reduce the expression efficiency of the polynucleotide encoding the human SMN protein in liver tissue; and/or
the human miRNA target sequence can reduce the expression efficiency of the polynucleotide encoding the human SMN protein in dorsal root ganglion tissue.

6. The gene expression cassette according to claim 5, wherein the human miRNA target sequence includes at least one of a miR-183 target sequence, a miR-182 target sequence, a miR-96 target sequence, a miR23b target sequence, a miR-145 target sequence, a miR-148a target sequence, a miR-22 target sequence, a miR-122 target sequence, a miR-143 target sequence, a miR-21 target sequence, or a miR-192 target sequence.

7. The gene expression cassette according to claim 5, wherein the human miRNA target sequence includes at least one of a miR-183 target sequence or a miR-122 target sequence.

8. The gene expression cassette according to claim 7, wherein the human miRNA target sequence includes the nucleotide sequence shown in any one of SEQ ID NOs: 21-22.

9. The gene expression cassette according to claim 7, wherein the number of the human miRNA target sequence is 1.

10. The gene expression cassette according to claim 7, wherein the number of the human miRNA target sequence is more than 1.

11. The gene expression cassette according to claim 10, wherein the human miRNA target sequence includes 2 miR-183 target sequences and 2 miR-122 target sequences.

12. The gene expression cassette according to claim 10, wherein the more than 1 human miRNA target sequence is operably linked via at least one spacer sequence, wherein the spacer sequence comprises the nucleotide sequence shown in any one of SEQ ID NOs: 4-6.

13. The gene expression cassette according to any one of claims 1-12, wherein the gene expression cassette further includes a promoter and a poly A sequence.

14. The gene expression cassette according to claim 13, wherein the promoter includes a CMV enhancer, a CBA promoter, a CAG promoter, a CBh promoter, an hSyn promoter, a CamKII promoter, an EF1α promoter, a UBC promoter, or an SMN endogenous promoter.

15. A recombinant vector, wherein the recombinant vector comprises the gene expression cassette according to any one of claims 1-14.

16. The recombinant vector according to claim 15, wherein the recombinant vector includes a recombinant plasmid expression vector or a recombinant viral vector.

17. The recombinant vector according to claim 16, wherein the recombinant viral vector includes a recombinant adenoviral vector, a recombinant adeno-associated viral vector, a recombinant retroviral vector, a recombinant herpes simplex viral vector, or a recombinant vaccinia viral vector.

18. The recombinant vector according to claim 17, wherein the recombinant viral vector is a recombinant adeno-associated viral vector.

19. The recombinant vector according to claim 18, wherein the serotype of the recombinant adeno-associated viral vector includes AAV1, AAV2, AAV3, AAV4, AAV5, AAV6, AAV7, AAV8, AAV9, AAVrh10, AAV11, AAV12, or AAV13.

20. The recombinant vector according to claim 19, wherein the serotype of the recombinant adeno-associated viral vector includes AAV1, AAV8, or AAV9.

21. The recombinant vector according to any one of claims 18-20, wherein the gene expression cassette is located downstream of the 5' inverted terminal repeat sequence of the adeno-associated viral vector.

22. A pharmaceutical composition, wherein the pharmaceutical composition includes the gene expression cassette according to any one of claims 1-14, or the recombinant vector according to any one of claims 15-21.

23. The pharmaceutical composition according to claim 22, wherein the form of the pharmaceutical composition includes an intraventricular injection drug, an intravenous injection drug, or an intrathecal injection drug.

24. An aqueous pharmaceutical formulation, wherein the aqueous pharmaceutical formulation includes the gene expression cassette according to any one of claims 1-14, or the recombinant vector according to any one of claims 15-21.

25. The aqueous pharmaceutical formulation according to claim 24, wherein the aqueous pharmaceutical formulation further includees 0.001% Poloxamer 188, 150 mM sodium chloride, 3 mM potassium chloride, 1.4 mM calcium chloride, 0.8 mM magnesium chloride, and 1 mM sodium phosphate buffer.

26. The aqueous pharmaceutical formulation according to claim 25, wherein the form of the aqueous pharmaceutical formulation includes an intraventricular injection drug, an intravenous injection drug, or an intrathecal injection drug.

27. Use of the gene expression cassette according to any one of claims 1-14, the recombinant vector according to any one of claims 15-21, the pharmaceutical composition according to any one of claims 22-23, or the aqueous pharmaceutical formulation according to any one of claims 24-26 for expressing a SMN protein in cells.

28. The use according to claim 27, wherein the cells are animal cells.

29. The use according to claim 28, wherein the animal cells include neuron.

30. Use of the gene expression cassette according to any one of claims 1-14, the recombinant vector according to any one of claims 15-21, the pharmaceutical composition according to any one of claims 22-23, or the aqueous pharmaceutical formulation according to any one of claims 24-26 in the preparation of a drug for preventing, alleviating, or treating a disease.

31. The use according to claim 30, wherein the disease is an SMN-related disease.

32. The use according to claim 31, wherein the SMN-related disease includes: a disease related to insufficient SMN protein expression, a disease related to SMN protein deficiency, a disease related to SMN1 gene deletion, or a disease related to SMN1 gene mutation.

33. The use according to claim 32, wherein the SMN-related disease is spinal muscular atrophy.

34. The use according to claim 33, wherein the spinal muscular atrophy includes SMA Type 1, SMA Type 2, SMA Type 3, or SMA Type 4.

35. The use according to claim 30, wherein the drug includes an intraventricular injection drug, an intravenous injection drug, or an intrathecal injection drug.

36. A method for preventing, alleviating, or treating a disease, including administering the gene expression cassette according to any one of claims 1-14, the recombinant vector according to any one of claims 15-21, the pharmaceutical composition according to any one of claims 22-23, or the aqueous pharmaceutical formulation according to any one of claims 24-26 to a subject in need thereof.

37. The method according to claim 36, wherein the disease is an SMN-related disease.

38. The method according to claim 37, wherein the SMN-related disease includes: a disease related to insufficient SMN protein expression, a disease related to SMN protein deficiency, a disease related to SMN1 gene deletion, or a disease related to SMN1 gene mutation.

39. The method according to claim 38, wherein the SMN-related disease is spinal muscular atrophy.

40. The method according to claim 39, wherein the spinal muscular atrophy includes SMA Type 1, SMA Type 2, SMA Type 3, or SMA Type 4.
